# EUROPEAN PATENT SPECIFICATION

(11) **EP 3 892 214 B1**
(45) Date of publication and mention of the grant of the patent: **03.04.2024**
(21) Application number: 20197917.6
(22) Date of filing: 23.09.2020
(51) Int. Cl.: A61B 17/34, A61B 90/10, A61B 90/11, A61B 17/00, A61M 25/00, A61M 25/01, A61B 5/055, A61B 90/00, A61B 5/06, G01R 33/28

(54) **MRI SURGICAL SYSTEMS INCLUDING MRI-COMPATIBLE SURGICAL CANNULAS FOR TRANSFERRING A SUBSTANCE TO AND/OR FROM A PATIENT**
CHIRURGISCHE MRT-SYSTEME MIT MRT-KOMPATIBLEN CHIRURGISCHEN KANÜLEN ZUR ÜBERTRAGUNG EINER SUBSTANZ IN DEN ODER AUS DEM KÖRPER EINES PATIENTEN
SYSTÈMES CHIRURGICAUX D'IRM COMPRENANT DES CANULES CHIRURGICALES COMPATIBLES IRM POUR LE TRANSFERT D'UNE SUBSTANCE SUR ET/OU DEPUIS UN PATIENT

(30) Priority: 08.04.2020 US 202063006929 P; 15.09.2020 US 202017021773
(43) Date of publication of application: 13.10.2021
(73) Proprietor: ClearPoint Neuro, Inc., Solana Beach, CA 92075 (US)
(72) Inventor: PIFERI, Peter, Orange, CA California 92867 (US)
(74) Representative: Yeadon IP Limited

(56) References cited:
- EP-A2- 2 558 154
- US-A1- 2017 056 617

## Description

### RELATED APPLICATION(S)

The present application claims the benefit of and priority from U.S. Provisional Patent Application No. 63/006,929, filed April 8, 2020.

### FIELD OF THE INVENTION

The present invention relates to MRI-guided diagnostic or interventional systems that may be particularly suitable for placement/localization of therapies in the body.

### BACKGROUND OF THE INVENTION

Various therapeutic and diagnostic procedures require that a substance be delivered (*e.g*., dispensed or infused) into a prescribed region of a patient, such as into a target deep brain location in the patient's brain, using a delivery cannula. It is often important or critical that the substance be delivered with high accuracy to the target region in the patient and without undue trauma to the patient.

EP2 558 154 B1 discloses a cannula for transferring a substance to and/or from a patient, the cannula comprising: a rigid tubular support sleeve and a transfer tube.

### SUMMARY

The cannula for transferring a substance to and/or from a patient according to the invention is defined in claim 1. Preferred embodiments are defined in the dependent claims. The surgical methods disclosed are not covered by the claimed invention.

According to some embodiments, a cannula for transferring a substance to and/or from a patient includes a rigid, tubular support sleeve, a transfer tube, and a conformal polymeric sleeve. The support sleeve defines a support sleeve lumen extending from a first opening at a proximal end of the support sleeve to a second opening at a distal end of the support sleeve. The support sleeve comprises a rigid, MRI-compatible material. The transfer tube is disposed in the support sleeve lumen and extends beyond the distal end of the support sleeve to a distal end of the transfer tube. The transfer tube defines a transfer tube lumen terminating at an opening at the distal end of the transfer tube. The conformal polymeric sleeve includes a proximal section surrounding at least a portion of the support sleeve and a transitional section connected to the proximal section and surrounding at least a portion of the transfer tube. The transitional section begins at the distal end of the support sleeve and extends from there in a direction toward the distal end of the transfer tube and over the transfer tube. a The transitional section tapers inwardly in the direction toward the distal end of the transfer tube.

In some embodiments, the transitional section is frustoconical.

According to some embodiments, the transitional section extends from the distal end of the support sleeve toward the distal end of the transfer tube an axial distance in the range of from about 0.1 mm to 5 mm.

In some embodiments, the support sleeve comprises a ceramic material.

In some embodiments, the conformal polymeric sleeve is a polymeric shrink tubing.

In some embodiments, the transfer tube is formed of fused silica.

According to some embodiments, the transfer tube is adhered by adhesive to an inner surface of the support sleeve.

In some embodiments, the conformal polymeric sleeve defines an annular void between an outer surface of the transfer tube and the transitional section of the conformal polymeric sleeve adjacent the distal end of the support sleeve, and the cannula includes an adhesive at least partially filling the annular void.

In some embodiments, the conformal polymeric sleeve extends to the distal end of the transfer tube.

In some embodiments, the conformal polymeric sleeve has a distal end located axially between the distal end of the support sleeve and the distal end of the transfer tube.

According to some embodiments, the cannula includes an inner sleeve disposed in the support sleeve lumen and extending beyond the distal end of the support sleeve to a distal end of the inner sleeve. The inner sleeve defines an inner sleeve lumen. The transfer tube is disposed in the inner sleeve lumen and extends to or beyond the distal end of the inner sleeve to the distal end of the transfer tube. The conformal polymeric sleeve surrounds at least a portion of the inner sleeve. The transitional section extends from the distal end of the support sleeve and over the inner sleeve in a direction toward the distal end of the inner sleeve. The transitional section tapers inwardly in the direction toward the distal end of the inner sleeve.

In some embodiments, the conformal polymeric sleeve defines an annular void between an outer surface of the inner sleeve and the transitional section of the conformal polymeric sleeve adjacent the distal end of the support sleeve, and the cannula includes an adhesive at least partially filling the annular void.

In some embodiments, the conformal polymeric sleeve extends to the distal end of the inner sleeve.

In some embodiments, the conformal polymeric sleeve covers a terminal end face of the inner sleeve at the distal end of the inner sleeve.

In some embodiments, the transfer tube extends axially beyond the inner sleeve in the direction of the distal end of the transfer tube.

According to some embodiments, the distal end of the inner sleeve is coterminous with the distal end of the transfer tube.

In some embodiments, the conformal polymeric sleeve extends to the distal end of the inner sleeve.

In some embodiments, the conformal polymeric sleeve has a distal end located axially between the distal end of the support sleeve and the distal end of the inner sleeve.

According to some embodiments, the transfer tube extends beyond the distal end of the inner sleeve. An exterior surface of the cannula has at least first, second and third co-axially disposed segments, an outer diameter of the second segment being greater than an outer diameter of the first segment, and an outer diameter of the third segment being greater than the outer diameter of the second segment. The second segment extends between and adjoins each of the first and third segments. The first segment extends from the distal end of the transfer tube to the distal end of the inner sleeve. The second segment extends from the distal end of the inner sleeve to a distal end of the transitional section of the conformal polymeric sleeve. The third segment extends from a proximal end of the transitional section toward the proximal end of the support sleeve. A terminal end face of the inner sleeve forms a sharp step about the transfer tube between the first segment and the second segment, the terminal end face of the inner sleeve projecting radially outwardly beyond the outer diameter of the first segment. The transitional section defines a second step between the second segment and the third segment, the second step having a smooth rise from the outer diameter of the second segment to the outer diameter of the third segment.

According to some embodiments, the inner sleeve is adhered with adhesive to an inner surface of the tubular support sleeve.

According to some embodiments, the inner sleeve is adhered with adhesive to an outer surface of the transfer tube.

According to some embodiments of the disclosure, not forming part of the claimed invention, a method of transferring a substance to and/or from a patient includes providing a cannula including: a rigid, tubular support sleeve defining a support sleeve lumen extending from a first opening at a proximal end of the support sleeve to a second opening at a distal end of the support sleeve, wherein the support sleeve comprises a rigid, MRI-compatible material; a transfer tube disposed in the support sleeve lumen and extending beyond the distal end of the support sleeve to a distal end of the transfer tube, the transfer tube defining a transfer tube lumen terminating at an opening at the distal end of the transfer tube; and a conformal polymeric sleeve surrounding at least a portion of the support sleeve and at least a portion of the transfer tube. The conformal polymeric sleeve includes a transitional section extending from the distal end of the support sleeve and over the transfer tube in a direction toward the distal end of the transfer tube. The transitional section tapers inwardly in the direction toward the distal end of the transfer tube. The method further includes: inserting the cannula into a selected region in the patient; and transferring the substance to or from the selected region through the transfer tube lumen.

In some embodiments of the disclosure, not forming part of the claimed invention, the method includes: partially withdrawing the cannula from a patient tissue in the selected region, thereby forming a channel in the patient tissue; and delivering stem cells through the cannula into the channel.

In some embodiments of the disclosure, not forming part of the claimed invention, the selected region is the brain.

According to some embodiments of the disclosure, a cannula for transferring a substance to and/or from a patient includes a rigid, tubular support sleeve, an inner sleeve, a transfer tube, and a conformal polymeric sleeve. The support sleeve defines a support sleeve lumen extending from a first opening at a proximal end of the support sleeve to a second opening at a distal end of the support sleeve. The support sleeve comprises a rigid, MRI-compatible material. The inner sleeve is disposed in the support sleeve lumen and extending beyond the distal end of the support sleeve to a distal end of the inner sleeve. The inner sleeve defines an inner sleeve lumen. The transfer tube is disposed in the inner sleeve lumen and extending to or beyond a distal end of the inner sleeve to a distal end of the transfer tube. The transfer tube defines a transfer tube lumen terminating at an opening at the distal end of the transfer tube. The conformal polymeric sleeve surrounds at least a portion of the support sleeve and at least a portion of the inner sleeve. The distal end of the inner sleeve is coterminous with the distal end of the transfer tube.

In some embodiments, the conformal polymeric sleeve extends to the distal end of the inner sleeve.

In some embodiments, the conformal polymeric sleeve has a distal end located axially between the distal end of the support sleeve and the distal end of the inner sleeve.

The support sleeve may comprise a ceramic material.

According to some embodiments, the conformal polymeric sleeve is a polymeric shrink tubing.

In some embodiments, the transfer tube is formed of fused silica.

According to some embodiments of the disclosure, not forming part of the claimed invention, a method of transferring a substance to and/or from a patient includes providing a cannula including: a rigid, tubular support sleeve defining a support sleeve lumen extending from a first opening at a proximal end of the support sleeve to a second opening at a distal end of the support sleeve, wherein the support sleeve comprises a rigid, MRI-compatible material; an inner sleeve disposed in the support sleeve lumen and extending beyond the distal end of the support sleeve to a distal end of the inner sleeve, the inner sleeve defining an inner sleeve lumen; a transfer tube disposed in the inner sleeve lumen and extending to or beyond a distal end of the inner sleeve to a distal end of the transfer tube, the transfer tube defining a transfer tube lumen terminating at an opening at the distal end of the transfer tube; and a conformal polymeric sleeve surrounding at least a portion of the support sleeve and at least a portion of the inner sleeve; wherein the distal end of the inner sleeve is coterminous with the distal end of the transfer tube. The method further includes: inserting the cannula into a selected region in the patient; and transferring the substance to or from the selected region through the transfer tube lumen.

In some embodiments of the disclosure, not forming part of the claimed invention, the method includes: partially withdrawing the cannula from a patient tissue in the selected region, thereby forming a channel in the patient tissue; and delivering stem cells through the cannula into the channel.

According to some embodiments of the disclosure, not forming part of the claimed invention, the selected region is the brain.

According to some embodiments of the disclosure, a cannula for transferring a substance to and/or from a patient includes a rigid, tubular support sleeve, a transfer tube, and a conformal polymeric sleeve. The support sleeve defines a support sleeve lumen extending from a first opening at a proximal end of the support sleeve to a second opening at a distal end of the support sleeve. The support sleeve comprises a rigid, MRI-compatible material. The transfer tube is disposed in the support sleeve lumen and extending beyond the distal end of the support sleeve to a distal end of the transfer tube. The transfer tube defines a transfer tube lumen terminating at an opening at the distal end of the transfer tube. The conformal polymeric sleeve surrounds at least a portion of the support sleeve and at least a portion of the transfer tube. The transfer tube is adhered by adhesive to an inner surface of the support sleeve.

In some embodiments, the conformal polymeric sleeve extends to the distal end of the transfer tube.

In some embodiments, the conformal polymeric sleeve has a distal end located axially between the distal end of the support sleeve and the distal end of the transfer tube.

According to some embodiments, the support sleeve comprises a ceramic material.

According to some embodiments, the conformal polymeric sleeve is a polymeric shrink tubing.

In some embodiments, the transfer tube is formed of fused silica.

According to some embodiments of the disclosure, not forming part of the claimed invention, a method of transferring a substance to and/or from a patient includes providing a cannula including: a rigid, tubular support sleeve defining a support sleeve lumen extending from a first opening at a proximal end of the support sleeve to a second opening at a distal end of the support sleeve, wherein the support sleeve comprises a rigid, MRI-compatible material; a transfer tube disposed in the support sleeve lumen and extending beyond the distal end of the support sleeve to a distal end of the transfer tube, the transfer tube defining a transfer tube lumen terminating at an opening at the distal end of the transfer tube; and a conformal polymeric sleeve surrounding at least a portion of the support sleeve and at least a portion of the transfer tube; wherein the transfer tube is adhered by adhesive to an inner surface of the support sleeve. The method further includes: inserting the cannula into a selected region in the patient; and transferring the substance to or from the selected region through the transfer tube lumen.

In some embodiments of the disclosure, not forming part of the claimed invention, the method includes: partially withdrawing the cannula from a patient tissue in the selected region, thereby forming a channel in the patient tissue; and delivering stem cells through the cannula into the channel.

According to some embodiments of the disclosure, not forming part of the claimed invention, the selected region is the brain.

### BRIEF DESCRIPTION OF THE DRAWINGS

**FIG. 1** is a schematic illustration of a MRI-guided surgical system.
**FIG. 2** is a side perspective view of a trajectory guide forming a part of the MRI-guided surgical system of **FIG. 1** mounted on a patient.
**FIG. 3** is a side perspective view of a part of the MRI-guided surgical system of **FIG. 1** mounted on the patient.
**FIG. 4** is an enlarged, perspective view of the trajectory guide of **FIG. 2****.**
**FIG. 5** is a sectional view of the trajectory guide of **FIG. 2** with a surgical cannula forming a part of the MRI-guided surgical system.
**FIGS. 6A-6C** and **7A-7B** are schematic illustrations of exemplary screen shots of displays of a User Interface provided to a user to facilitate navigation and/or assessment steps of an MRI-guided substance transfer (*e.g*., delivery) procedure.
**FIG. 8A** is a side view of a surgical cannula according to further embodiments of the present disclosure.
**FIG. 8B** is a cross-sectional view of the surgical cannula of **FIG. 8A** taken along the line **8B-8B** of **FIG. 8A****.**
**FIG. 8C** is an enlarged, cross-sectional view of Detail **8C** of **FIG. 8B****.**
**FIG. 8D** is an enlarged, cross-sectional view of Detail **8D** of **FIG. 8B****.**
**FIG. 8E** is an enlarged, cross-sectional view of Detail **8E** of **FIG. 8B****.**
**FIG. 8F** is a further enlarged, cross-sectional view of Detail **8E** of **FIG. 8B****.**
**FIG. 9A** is a fragmentary, cross-sectional view of a surgical cannula according to further embodiments of the present disclosure.
**FIG. 9B** is an enlarged, fragmentary, cross-sectional view of a midsection of the surgical cannula **FIG. 9A****.**
**FIG. 9C** is an enlarged, fragmentary, cross-sectional view of a tip section of the surgical cannula **FIG. 9A****.**
**FIG. 10A** is a fragmentary, cross-sectional view of a surgical cannula according to further embodiments of the present disclosure.
**FIG. 10B** is an enlarged, fragmentary, cross-sectional view of a midsection of the surgical cannula **FIG. 10A****.**
**FIG. 10C** is an enlarged, fragmentary, cross-sectional view of a tip section of the surgical cannula **FIG. 10A****.**
**FIG. 11A** is a fragmentary, cross-sectional view of a surgical cannula according to further embodiments of the present invention.
**FIG. 11B** is an enlarged, fragmentary, cross-sectional view of a midsection of the surgical cannula **FIG. 11A****.**
**FIG. 11C** is an enlarged, fragmentary, cross-sectional view of a tip section of the surgical cannula **FIG. 11A****.**
**FIG. 12A** is a fragmentary, cross-sectional view of a surgical cannula according to further embodiments of the present disclosure.
**FIG. 12B** is an enlarged, fragmentary, cross-sectional view of a midsection of the surgical cannula **FIG. 12A****.**
**FIG. 13** is a data processing system according to some embodiments of the present disclosure.

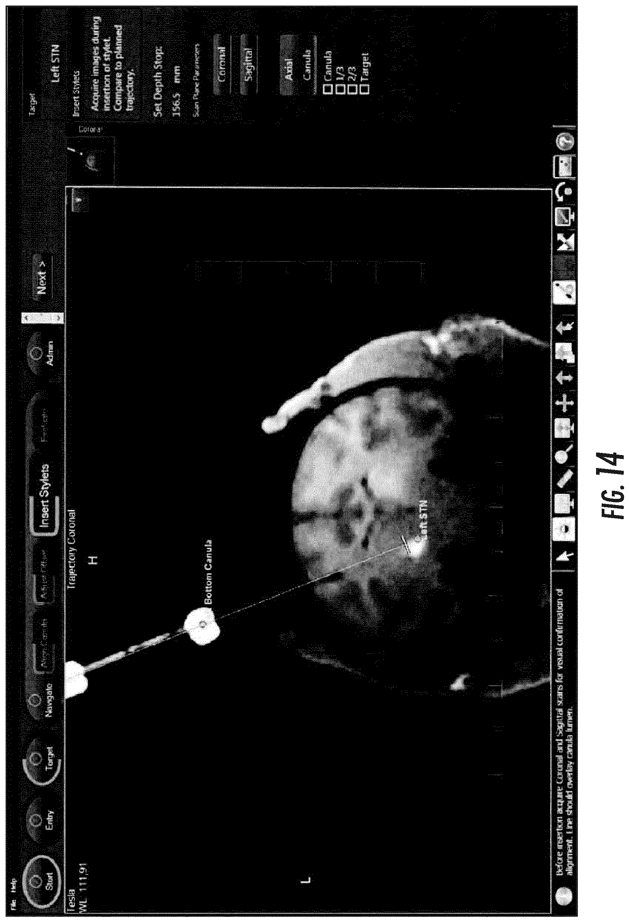

**FIG. 14** is an exemplary screen shot of a display of a User Interface during an MRI-guided substance delivery procedure.

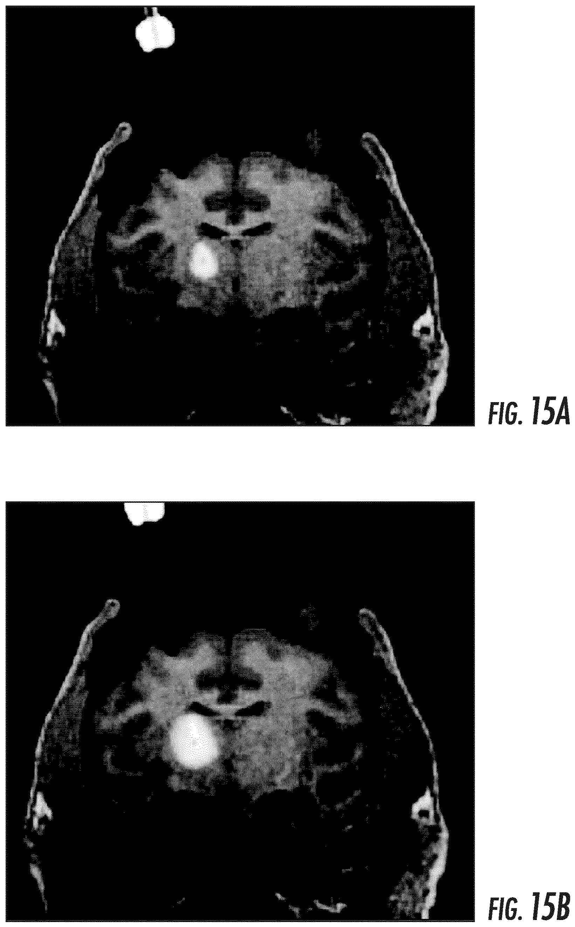

**FIGS. 15A** and **15B** are exemplary screen shots of a display of a User Interface during an MRI-guided substance delivery procedure and captured at a first time and a second, subsequent time, respectively.

### DETAILED DESCRIPTION OF EMBODIMENTS OF THE INVENTION

The present invention will now be described more fully hereinafter with reference to the accompanying drawings, in which embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. Like numbers refer to like elements throughout. It will be appreciated that although discussed with respect to a certain embodiment, features or operation of one embodiment can apply to others.

In the drawings, the thickness of lines, layers, features, components and/or regions may be exaggerated for clarity and broken lines (such as those shown in circuit of flow diagrams) illustrate optional features or operations, unless specified otherwise.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

It will be understood that when a feature, such as a layer, region or substrate, is referred to as being "on" another feature or element, it can be directly on the other element or intervening elements may also be present. In contrast, when an element is referred to as being "directly on" another feature or element, there are no intervening elements present. It will also be understood that, when a feature or element is referred to as being "connected" or "coupled" to another feature or element, it can be directly connected to the other element or intervening elements may be present. In contrast, when a feature or element is referred to as being "directly connected" or "directly coupled" to another element, there are no intervening elements present. Although described or shown with respect to one embodiment, the features so described or shown can apply to other embodiments.

The term "electroanatomical visualization" refers to a visualization or map of the anatomical structure, *e.g*., brain, typically a volumetric, 3-D map or 4-D map, that illustrates or shows electrical activity of tissue correlated to anatomical and/or coordinate spatial position. The visualization can be in color and color-coded to provide an easy to understand map or image with different measures or gradients of activity in different colors and/or intensities. The term "color-coded" means that certain features, electrical activity or other output are shown with defined colors of different color and/or intensity to visually accentuate different tissue, different and similar electrical activity or potential in tissue and/or to show abnormalities or lesions in tissue versus normal or non-lesion tissue. In some embodiments, the systems can be configured to allow a clinician to increase or decrease the intensity or change a color of certain tissue types or electrical outputs, *e.g.,* in high-contrast color and/or intensity, darker opacity or the like.

The actual visualization can be shown on a screen or display so that the map and/or anatomical or tool structure is in a flat 2-D view and/or in 2-D what appears to be 3-D volumetric images with data representing features or electrical output with different visual characteristics such as with differing intensity, opacity, color, texture and the like. For example, the 3-D image of the lung can be generated to illustrate differences in barrier thickness using color or opacity differences over the image volume. Thus, the term "3-D" in relation to images does not require actual 3-D viewability (such as with 3-D glasses), just a 3-D appearance, typically on a display. The 3-D images comprise multiple 2-D slices. The 3-D images can be volume renderings well known to those of skill in the art and/or a series of 2-D slices, which can be visually paged through. A 4-D map illustrates time-dependent activity, such as electrical activity or blood flow movement.

The surgical systems may be configured to operate based on known physical characteristics of one or more surgical tools, which may include a surgical (*e.g*., delivery) cannula, such that the hardware is a point of interface for the circuit or software. The systems can communicate with databases that define dimensions, configurations or shapes and spacing of components on the tool(s). The defined physical data can be obtained from a CAD model of a tool. The physical characteristics can include dimensions or other physical features or attributes and may also include relative changes in position of certain components or features upon a change in position of a tool or portion thereof. The defined physical characteristics can be electronically (programmatically) accessible by the system or known *a priori* and electronically stored locally or remotely and used to automatically calculate certain information and/or to segment image data. That is, tool data from the known dimensions and configuration of the tool model can be used to segment image data and/or correlate a position and orientation of a tool and/or provide trajectory adjustment guidelines or error estimates, warnings of improper trajectories and the like. For example, the system can include defined structural and/or operational details/data for one or more of a delivery cannula, a grid for marking a burr hole location and/or a trajectory guide. The system can use this data to allow a user to adjust an intrabrain path for placing a diagnostic or therapy device. Such can be input, transposed, and/or overlayed in a visualization of the tool on one or more displays along with patient structure or otherwise used, such as, for example, to project the information onto a patient's anatomical structure or determine certain operational parameters including which image volume (scan planes) to use to obtain MRI image data that will include select portions of the targeting cannula or surgical cannula. As such, at least some of the generated visualizations are not merely an MRI image of the patient during a procedure.

The visualizations are rendered visualizations that can combine multiple sources of data to provide visualizations of spatially encoded tool position and orientation with anatomical structure and can be used to provide position adjustment data output so that a clinician can obtain a desired trajectory path, thereby providing a smart-adjustment system without requiring undue "guess" work on what adjustments to make to obtain the desired trajectory.

The term "animation" refers to a sequence or series of images shown in succession, typically in relatively quick succession, such as in about 1-50 frames per second. The term "frame" refers to a single visualization or static image. The term "animation frame" refers to one image frame of the different images in the sequence of images.

The term "ACPC coordinate space" refers to a right-handed coordinate system defined by anterior and posterior commissures (AC, PC) and Mid-Sagittal plane points, with positive directions corresponding to a patient's anatomical Right, Anterior and Head directions with origin at the mid-commissure point.

The term "grid" refers to a pattern of crossed lines or shapes used as a reference for locating points or small spaces, *e.g.,* a series of rows and intersecting columns, such as horizontal rows and vertical columns (but orientations other than vertical and horizontal can also be used). The grid can include associated visual indicia such as alphabetical markings (*e.g*., A-Z and the like) for rows and numbers for columns (*e.g*., 1-10) or the reverse. Other marking indicia may also be used. The grid can be provided as a flexible patch that can be releasably attached to the skull of a patient. For additional description of suitable grid devices, *see* copending, co-assigned U.S. Patent Application Serial No. 12/236,621 (U.S. Published Patent Application No. US 2009/0177077 A1).

The term "fiducial marker" refers to a marker that can be electronically identified using image recognition and/or electronic interrogation of MRI image data. The fiducial marker can be provided in any suitable manner, such as, but not limited to, a geometric shape of a portion of the tool, a component on or in the tool, a coating or fluid-filled component or feature (or combinations of different types of fiducial markers) that makes the fiducial marker(s) MRI-visible with sufficient signal intensity (brightness) or generates a "void" or dark space for identifying location and/or orientation information for the tool and/or components thereof in space.

The term "MRI scanner" refers to a magnetic resonance imaging and/or NMR spectroscopy system. As is well known, MRI scanners include a low field strength magnet (typically between about .1T to about .5T), a medium field strength magnet, or a high-field strength super-conducting magnet, an RF pulse excitation system, and a gradient field system. MRI scanners are well known to those of skill in the art. Examples of commercially available clinical MRI scanners include, for example, those provided by General Electric Medical Systems, Siemens, Philips, Varian, Bruker, Marconi, Hitachi and Toshiba. The MRI systems can be any suitable magnetic field strength, such as, for example, about 1.5T or about 3.0T, and may include other high-magnetic field systems between about 2.0T-10.0T.

The term "RF safe" means that the lead or probe is configured to safely operate when exposed to RF signals, particularly RF signals associated with MRI systems, without inducing unplanned current that inadvertently unduly heats local tissue or interferes with the planned therapy.

The term "MRI visible" means that the device is visible, directly or indirectly, in an MRI image. The visibility may be indicated by the increased SNR of the MRI signal proximate the device.

The term "MRI compatible" means that the so-called component(s) is suitable for use in an MRI environment and as such is typically made of a non-ferromagnetic MRI compatible material(s) suitable to reside and/or operate in or proximate a conventional medical high magnetic field environment. The "MRI compatible" component or device is "MR safe" when used in the MRI environment and has been demonstrated to neither significantly affect the quality of the diagnostic information nor have its operations affected by the MR system at the intended use position in an MR system. These components or devices may meet the standards defined by ASTM F2503-05. *See,* American Society for Testing and Materials (ASTM) International, Designation: F2503-05. Standard Practice for Marking Medical Devices and Other Items for Safety in the Magnetic Resonance Environment. ASTM International, West Conshohocken, PA, 2005.

The term "near real time" refers to both low latency and high frame rate. Latency is generally measured as the time from when an event occurs to display of the event (total processing time). For tracking, the frame rate can range from between about 100 fps to the imaging frame rate. In some embodiments, the tracking is updated at the imaging frame rate. For near 'real-time' imaging, the frame rate is typically between about 1 fps to about 20 fps, and in some embodiments, between about 3 fps to about 7 fps. The low latency required to be considered "near real time" is generally less than or equal to about 1 second. In some embodiments, the latency for tracking information is about 0.01s, and typically between about 0.25-0.5s when interleaved with imaging data. Thus, with respect to tracking, visualizations with the location, orientation and/or configuration of a known intrabody device can be updated with low latency between about 1 fps to about 100 fps. With respect to imaging, visualizations using near real time MR image data can be presented with a low latency, typically within between about 0.01 ms to less than about 1 second, and with a frame rate that is typically between about 1-20 fps. Together, the system can use the tracking signal and image signal data to dynamically present anatomy and one or more intrabody devices in the visualization in near real-time. In some embodiments, the tracking signal data is obtained and the associated spatial coordinates are determined while the MR image data is obtained and the resultant visualization(s) with the intrabody device (*e.g*., surgical cannula) and the near RT MR image(s) are generated.

The term "automatically" means that the operation can be substantially, and typically entirely, carried out without human or manual input, and is typically programmatically directed or carried out. The term "electronically" includes both wireless and wired connections between components. The term "programmatically" means under the direction of a computer program that communicates with electronic circuits and other hardware and/or software.

The term "surgical cannula" refers to an intrabody cannula used to transfer a substance to and/or from a target intrabody location.

Embodiments of the invention may be particularly suitable for use with human patients but may also be used with any animal or other mammalian subject.

Embodiments of the present disclosure may take the form of an entirely software embodiment or an embodiment combining software and hardware aspects, all generally referred to herein as a "circuit" or "module." In some embodiments, the circuits include both software and hardware and the software is configured to work with specific hardware with known physical attributes and/or configurations. Furthermore, the present disclosure may take the form of a computer program product on a computer-usable storage medium having computer-usable program code embodied in the medium. Any suitable computer readable medium may be utilized including hard disks, CD-ROMs, optical storage devices, a transmission media such as those supporting the Internet or an intranet, or other storage devices.

Computer program code for carrying out operations of the present disclosure may be written in an object oriented programming language such as Java^{®}, Smalltalk or C++. However, the computer program code for carrying out operations of the present disclosure may also be written in conventional procedural programming languages, such as the "C" programming language. The program code may execute entirely on the user's computer, partly on the user's computer, as a stand-alone software package, partly on the user's computer and partly on another computer, local and/or remote or entirely on the other local or remote computer. In the latter scenario, the other local or remote computer may be connected to the user's computer through a local area network (LAN) or a wide area network (WAN), or the connection may be made to an external computer (for example, through the Internet using an Internet Service Provider).

Embodiments are described in part below with reference to flowchart illustrations and/or block diagrams of methods, apparatus (systems) and computer program products according to embodiments of the present disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function/act specified in the flowchart and/or block diagram block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions/acts specified in the flowchart and/or block diagram block or blocks.

The flowcharts and block diagrams of certain of the figures herein illustrate exemplary architecture, functionality, and operation of possible implementations of embodiments of the present disclosure. In this regard, each block in the flow charts or block diagrams represents a module, segment, or portion of code, which comprises one or more executable instructions for implementing the specified logical function(s). It should also be noted that in some alternative implementations, the functions noted in the blocks may occur out of the order noted in the figures. For example, two blocks shown in succession may in fact be executed substantially concurrently or the blocks may sometimes be executed in the reverse order or two or more blocks may be combined, depending upon the functionality involved.

Some embodiments of the present disclosure are directed to MRI-guided systems that can generate substantially real time (*e.g*., near real-time) patient-specific visualizations of the patient and one or more surgical tools, including an MRI-compatible intrabody surgical cannula (*e.g*., delivery cannula) and the delivery distribution, location, pattern, etc., in logical space and provide feedback to a clinician to improve the speed and/or reliability of an intrabody infusion or delivery of a substance to a target within the body through the delivery cannula. The delivery cannula has at least one lumen and at least one exit port configured to direct a flow of the substance through the lumen and the exit port to the target.

Some embodiments of the present disclosure are directed to MRI-guided systems that can generate substantially real time patient-specific visualizations of the patient and a distribution of a substance delivered to a target within the patient through an MRI-compatible delivery cannula in logical space and provide feedback to a clinician to improve the speed and/or reliability of an intrabody infusion or delivery of the substance. These systems can show a dynamic dispersion and/or infusion pattern of the substance delivered or dispensed into the patient. MRI can be effectively used to monitor the efficacy and/or delivery of the substance from the delivery cannula.

The visualizations can be based (in-part) on predefined data of the delivery cannula which can define a point of interface for the system (*e.g*., software) based on predefined characteristics of the delivery cannula, *e.g.,* dimensions, shape or configuration and/or known rotational, translational and/or other functional and/or dynamic behavior of the delivery cannula (*e.g*., flow rate, nozzle angle). The visualizations can include patient function data (*e.g*., fMRI data, electrical activity, active regions of a brain during a defined stimulation, fiber tracks, and the like).

The system can be configured to interrogate and segment image data to locate fiducial markers in the image (*e.g*., an increased higher intensity pixel/voxel region and/or void created in the MRI image by the presence of the delivery cannula in the patient's tissue) and generate successive visualizations of the patient's anatomical structure and the delivery cannula using MRI image data and *a priori* data of the delivery cannula to provide (substantially real-time) visualizations of the distribution of the substance in the patient.

Some embodiments of the present disclosure can provide visualizations to allow more precise control, delivery, and/or feedback of an infusion or delivery therapy so that the therapy or delivery cannula associated therewith can be more precisely placed and/or so that the cannula or delivery can be adjusted to provide the desired distribution in tissue, or to confirm proper delivery and allow near real-time visualization of the procedure.

Some embodiments of the present disclosure are directed to MRI-compatible intrabody delivery cannulas that may be used to precisely deliver any suitable and desired substance (*e.g*., cellular, biological, and/or drug therapeutics) to the desired anatomy target. The delivery cannulas can be used in and the systems can be configured to guide and/or place the delivery cannula in any desired internal region of the body of the patient, but may be particularly suitable for neurosurgeries and delivery of a substance to a target area or region within the brain. The delivery cannulas and systems can be used for gene and/or stem-cell based therapy delivery or other neural therapy delivery and allow user-defined custom targets in the brain or to other locations. Cannulas, systems and methods disclosed may be used to treat patients by delivery of cellular/biological therapeutics into the desired anatomy to modify their cellular function. The cells (*e.g*., stem cells) may improve function.

The target region may be any suitable region or area within the patient body. According to some embodiments, the target region is a STN anatomical region, which may be identified and located with reference to standard anatomical landmarks. According to some embodiments, the target area is a deep brain tumor or other undesirable tissue mass.

According to some embodiments, the target is intrathecal. The intrathecal target may be in the brain or spinal cord.

The substance delivered to the target region through the delivery cannula may be any suitable and desired substance. According to some embodiments, the substance is a liquid or slurry. In the case of a tumor, the substance may be a chemotherapeutic (cytotoxic) fluid. In some embodiments, the substance can include certain types of advantageous cells that act as vaccines or other medicaments (for example, antigen presenting cells such as dentritic cells). The dentritic cells may be pulsed with one or more antigens and/or with RNA encoding one or more antigen. Exemplary antigens are tumor-specific or pathogen-specific antigens. Examples of tumor-specific antigens include, but are not limited to, antigens from tumors such as renal cell tumors, melanoma, leukemia, myeloma, breast cancer, prostate cancer, ovarian cancer, lung cancer and bladder cancer. Examples of pathogen-specific antigens include, but are not limited to, antigens specific for HIV or HCV. In some embodiments, the substance may comprise radioactive material such as radioactive seeds. Substances delivered to a target area may include, but are not limited to, the following as shown in **Table 1:**

**Table 1**

| **DRUG (generic name)** | **DISORDER(S)** |
|---|---|
| caprylidene | Alzheimer's disease |
| donepezil | Alzheimer's disease |
| galantamine | Alzheimer's disease |
| memantine | Alzheimer's disease |
| Tacrine | Alzheimer's disease |
| vitamin E | Alzheimer's disease |
| ergoloid mesylates | Alzheimer's disease |
| riluzole | Amyotrophic lateral sclerosis |
| metoprolol | Benign essential tremors |
| primidone | Benign essential tremors |
| propanolol | Benign essential tremors |
| gabapentin | Benign essential tremors & Epilepsy |
| nadolol | Benign essential tremors & Parkinson's disease |
| zonisamide | Benign essential tremors & Parkinson's disease |
| carmustine | Brain tumor |
| lomustine | Brain tumor |
| methotrexate | Brain tumor |
| cisplatin | Brain tumor & Neuroblastoma |
| ioversol | Cerebral arteriography |
| mannitol | Cerebral Edema |
| dexamethasone | Cerebral Edema & Neurosarcoidosis |
| baclofen | Cerebral spasticity |
| ticlopidine | Cerebral thrombosis / embolism |
| isoxsuprine | Cerebrovascular insufficiency |
| cefotaxime | CNS infection & Meningitis |
| acyclovir | Encephalitis |
| foscarnet | Encephalitis |
| ganciclovir | Encephalitis |
| interferon alpha-2a | Encephalitis |
| carbamazepine | Epilepsy |
| clonazepam | Epilepsy |
| diazepam | Epilepsy |
| divalproex sodium | Epilepsy |
| ethosuximide | Epilepsy |
| ethotoin | Epilepsy |
| felbamate | Epilepsy |
| fosphenytoin | Epilepsy |
| levetiracetam | Epilepsy |
| mephobarbital | Epilepsy |
| paramethadione | Epilepsy |
| phenytoin | Epilepsy |
| trimethadione | Epilepsy |
| pregabalin | Epilepsy & Neuralgia |
| immune globulin intravenous | Guillain-Barre Syndrome |
| interferon beta-1b | Guillain-Barre Syndrome & Multiple sclerosis |
| azathioprine | Guillain-Barre Syndrome & Multiple sclerosis & Neurosarcoidosis |
| risperidone | Head injury |
| tetrabenazine | Huntington's disease |
| acetazolamide | Hydrocephalus & Epilepsy |
| alteplase | Ischemic stroke |
| clopidogrel | Ischemic stroke |
| nimodipine | Ischemic stroke & Subarachnoid hemorrhage |
| Aspirin | Ischemic stroke & Thromboembolic stroke |
| amikacin | Encaphalitis |
| ampicillin | Encaphalitis |
| ampicillin/sulbactam | Encaphalitis |
| ceftazidime | Encaphalitis |
| ceftizoxime | Encaphalitis |
| cefuroxime | Encaphalitis |
| chloramphenicol | Encaphalitis |
| cilastatin/imipenem | Encaphalitis |
| gentamicin | Encaphalitis |
| meropenem | Encaphalitis |
| metronidazole | Encaphalitis |
| nafcillin | Encaphalitis |
| oxacillin | Encaphalitis |
| piperacillin | Encaphalitis |
| rifampin | Encaphalitis |
| sulfamethoxazole/trimethoprim | Encaphalitis |
| tobramycin | Encaphalitis |
| triamcinolone | Encaphalitis |
| vancomycin | Encaphalitis |
| ceftriaxone | Encaphalitis & Neurosyphilis |
| pennicillin | Encaphalitis & Neurosyphilis |
| corticotropin | Multiple sclerosis |
| dalfampridine | Multiple sclerosis |
| glatiramer | Multiple sclerosis |
| mitoxantrone | Multiple sclerosis |
| natalizumab | Multiple sclerosis |
| modafinil | Multiple sclerosis |
| cyclophosphamide | Multiple sclerosis & Brain tumor & Neuroblastoma |
| interferon beta-1a | Multiple sclerosis & Neuritis |
| prednisolone | Multiple sclerosis & Neurosarcoidosis |
| prednisone | Multiple sclerosis & Neurosarcoidosis |
| amantadine | Multiple sclerosis & Parkinson's disease |
| methylprednisolone | Neuralgia |
| desvenlafaxine | Neuralgia |
| nortriptyline | Neuralgia |
| doxorubicin | Neuroblastoma |
| vincristine | Neuroblastoma |
| albendazole | Neurocystecercosis |
| chloroquine phosphate | Neurosarcoidosis |
| hydroxychloroquine | Neurosarcoidosis |
| infliximab | Neurosarcoidosis |
| pentoxyfilline | Neurosarcoidosis |
| thalidomide | Neurosarcoidosis |
| apomorphine | Parkinson's disease |
| belladonna | Parkinson's disease |
| benztropine | Parkinson's disease |
| biperiden | Parkinson's disease |
| bromocriptine | Parkinson's disease |
| carbidopa | Parkinson's disease |
| carbidopa/entacapone/levodopa | Parkinson's disease |
| carbidopa/levodopa | Parkinson's disease |
| entacapone | Parkinson's disease |
| levodopa | Parkinson's disease |
| pergolide mesylate | Parkinson's disease |
| pramipexole | Parkinson's disease |
| procyclidine | Parkinson's disease |
| rasagiline | Parkinson's disease |
| ropinirole | Parkinson's disease |
| rotiotine | Parkinson's disease |
| scopolamine | Parkinson's disease |
| tolcapone | Parkinson's disease |
| trihexyphenidyl | Parkinson's disease |
| seleginline | Parkinson's disease |
| rivastigmine | Parkinson's disease & Alzheimer's disease |
| anisindione | Thromboembolic stroke |
| warfarin | Thromboembolic stroke |
| 5-hydroxytryptophan | Depression & Anxiety & ADHD |
| duloxetine | Depression & Anxiety & Bipolar disorder |
| escitalopram | Depression & Anxiety & Bipolar disorder |
| | Depression & Anxiety & Bipolar disorder & Autism & |
| venlafaxine | Social anxiety disorder |
| desvenlafaxine | Depression & Anxiety & PTSD & ADHD |
| paroxetine | Depression & Anxiety & PTSD & Social anxiety disorder |
| fluoxetine/olanzapine | Depression & Bipolar disorder |
| 1-methylfolate | Depression & BPD |
| amitriptyline | Depression & PTSD |
| sertraline | Depression & PTSD & Bipolar disorder & Social anxiety disorder |
| fluvoxamine | Depression & PTSD & Social anxiety disorder |
| olanzapine | Depression & Schizophrenia & Bipolar disorder |
| paliperidone | Depression & Schizophrenia & Bipolar disorder |
| aripiprazole | Depression & Schizophrenia & Bipolar disorder & Autism |
| quetiapine | Depression & Schizophrenia & PTSD & BPD & Bipolar disorder |
| risperidone | Depression & Schizophrenia & PTSD & BPD & Bipolar disorder & Autism |
| amisulpride | Depression & Social anxiety disorder |
| chlorpromazine | Psychosis |
| droperidol | Psychosis |
| fluphenazine | Psychosis |
| periciazine | Psychosis |
| perphenazine | Psychosis |
| thiothixene | Psychosis |
| triflupromazine | Psychosis |
| haloperidol | Psychosis & Dementia |
| prazosin | PTSD |
| clozapine | Schizophrenia |
| flupenthixol | Schizophrenia |
| iloperidone | Schizophrenia |
| loxapine | Schizophrenia |
| mesoridazine | Schizophrenia |
| promazine | Schizophrenia |
| reserpine | Schizophrenia |
| thioridazein | Schizophrenia |
| zuclopenthixol | Schizophrenia |
| asenapine | Schizophrenia & Bipolar disorder |
| levomepromazine | Schizophrenia & Bipolar disorder |
| ziprasidone | Schizophrenia & Bipolar disorder |
| molindone | Schizophrenia & Psychosis |
| pimozide | Schizophrenia & Psychosis |
| thioridazine | Schizophrenia & Psychosis |
| Cytarabine | Chemotherapy, hematological malignancies |

According to some embodiments, the surgical cannula is used to remove or withdraw a substance therethrough from the target area. According to some embodiments, the surgical cannula is used to remove cerebral spinal fluid from the patient.

Embodiments of the present disclosure may include steps, features, aspects, components, procedures and/or systems as disclosed in U.S. Published Patent Application No. 2009/0171184, and PCT Published Patent Application No. WO 2011/130107 A2.

According to some embodiments, the systems are configured to provide a substantially automated or semi-automated and relatively easy-to-use MRI-guided system with defined workflow steps and interactive visualizations. In particular embodiments, the systems define and present workflow with discrete steps for finding target and entry point(s), guiding the alignment of the targeting cannula to a planned trajectory, monitoring the insertion of the delivery cannula, and adjusting the (X-Y) position in cases where the placement needs to be corrected. During steps where specific MR scans are used, the circuit or computer module can display data for scan plane center and angulation to be entered at the console. The workstation/circuit can passively or actively communicate with the MR scanner. The system can also be configured to use functional patient data (*e.g*., fiber tracks, fMRI and the like) to help plan or refine a target surgical site and/or access path.

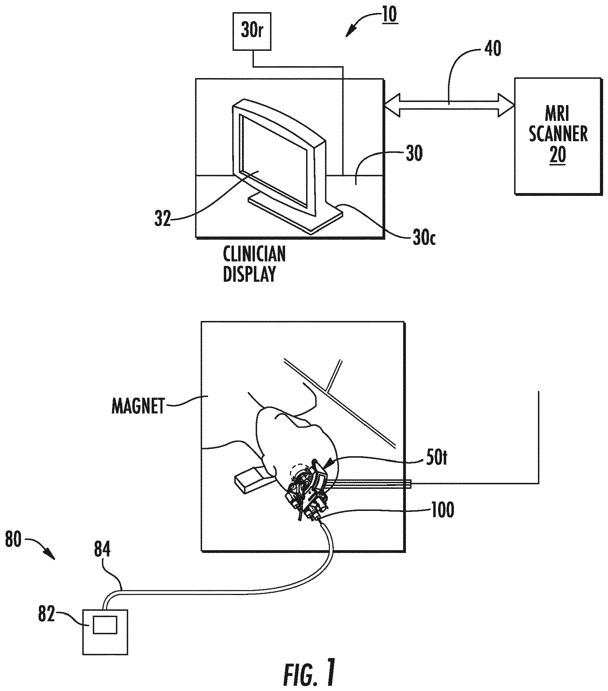

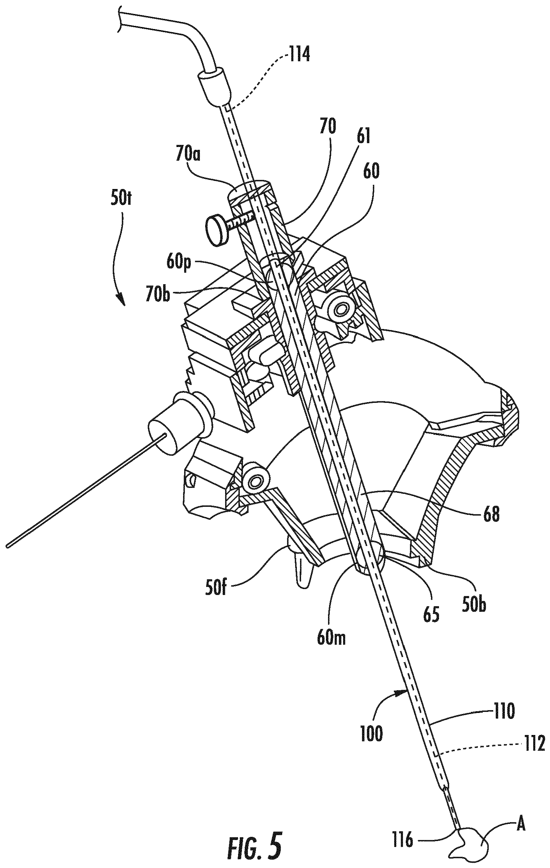

Embodiments of the present disclosure will now be described in further detail below with reference to the figures. **FIG. 1** illustrates an MRI-guided interventional system **10** with an MRI scanner **20,** a clinician workstation **30** with at least one circuit **30c,** at least one display **32,** an MRI compatible trajectory guide **50t,** a depth stop **70 (****FIG. 5****),** and a fluid substance delivery system **80.** The fluid substance delivery system includes an MRI-compatible intrabody surgical or delivery cannula **100,** an infusion or delivery pump **82** and connecting tubing **84.** The system **10** can be configured to render or generate real time visualizations of the target anatomical space using MRI image data and predefined data of at least one surgical tool to segment the image data and place the trajectory guide **50t** and the cannula **100** in the rendered visualization in the correct orientation and position in 3D space, anatomically registered to a patient. The trajectory guide **50t** and the cannula **100** can include or cooperate with tracking, monitoring and/or interventional components.

The tools of the system **10,** including the cannula **100,** can be provided as a sterile kit (typically as single-use disposable hardware) or in other groups or sub-groups or even individually, typically provided in suitable sterile packaging. The tools can also include a marking grid (*e.g*., as disclosed in U.S. Published Patent Application No. 2009/0177077 and/or U.S. Published Patent Application No. 2009/0171184). Certain components of the kit may be replaced or omitted depending on the desired procedure. Certain components can be provided in duplicate for bilateral procedures.

With reference to **FIG. 5****,** the depth stop **70** has a generally cylindrical configuration with opposite proximal and distal ends **70a, 70b** and is adapted to be removably secured within the proximal end of the tubular trajectory guide member **50t.** The depth stop **70** can be attached to the cannula **100** to allow for a defined insertion depth where insertion depth control and/or locking, is desired.

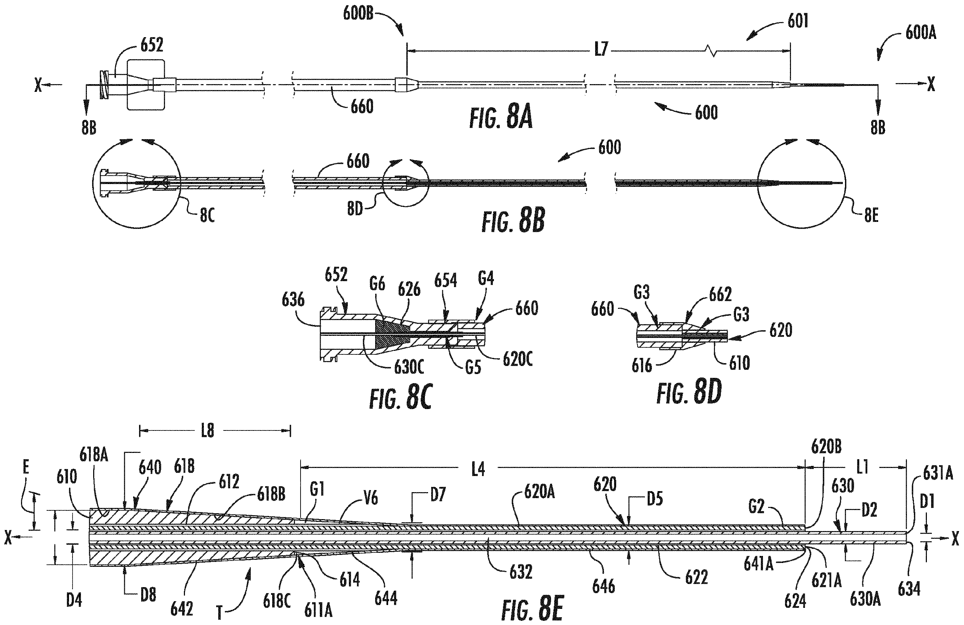

The cannula **100** can be configured to flowably introduce and/or inject a desired therapy (*e.g*., antigen, gene therapy, chemotherapy or stem-cell or other therapy type). The cannula **100** as shown in **FIG. 5** includes a cannula body **110** defining at least one longitudinally extending lumen **112,** a first or inlet port **114** and at least one second or exit port **116.** The cannula **100** is formed of an MRI-compatible, MRI-visible material such as ceramic. The cannula **100** as depicted in **FIG. 5** is a relatively simple embodiment and further embodiments including additional functionality will be disclosed hereinbelow with reference to **FIGS. 8A-****12B.** These further embodiments of cannula may be used in the same manner as described with regard to the cannula **100.**

The lumen **112** can be fluidly connected to the pump **82** by the tubing **84.** The tubing **84** may be flexible tubing. According to some embodiments, the tubing **84** is PVC tubing. According to some embodiments, the tubing **84** is silicone tubing. In some embodiments, the cannula can be self-loading and not require tubing and/or a pump.

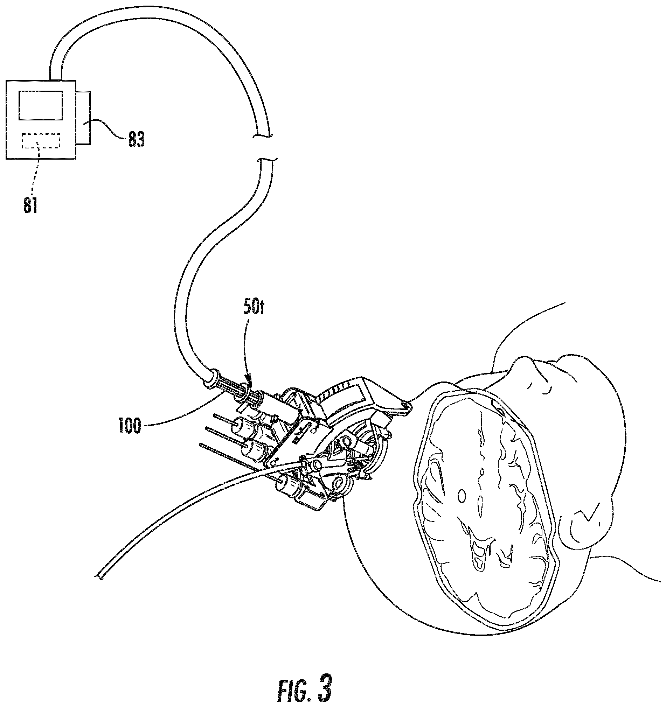

According to some embodiments and with reference to **FIG. 3****,** the pump **82** includes a reservoir **83** of the substance to be delivered and a pump mechanism **81.** The pump mechanism **81** is selectively operable to supply the substance to the lumen **112** under a controlled pressure. According to some embodiments, a syringe (*e.g.,* a hand syringe) is used in place of the pump **82.**

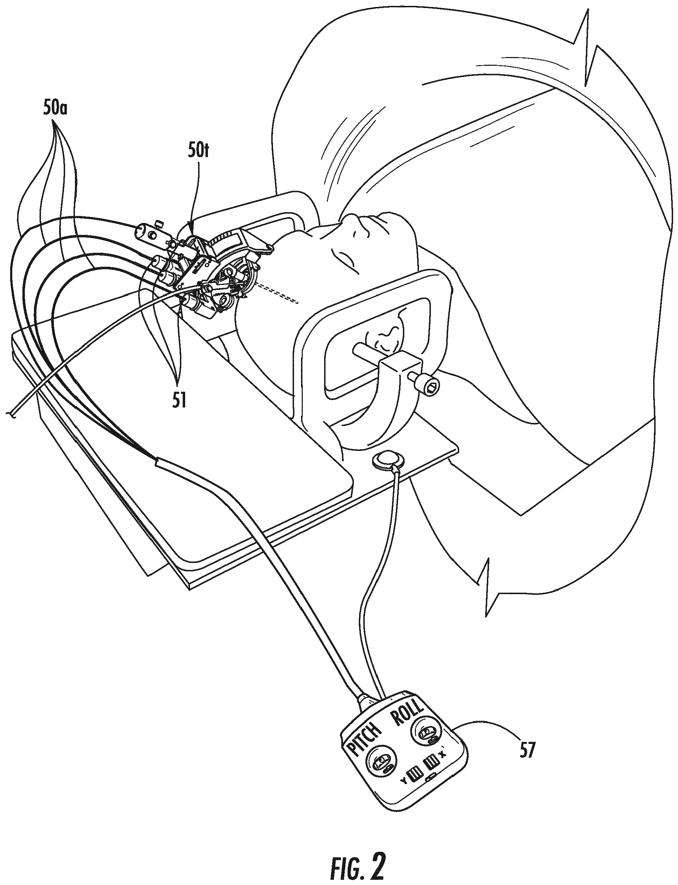

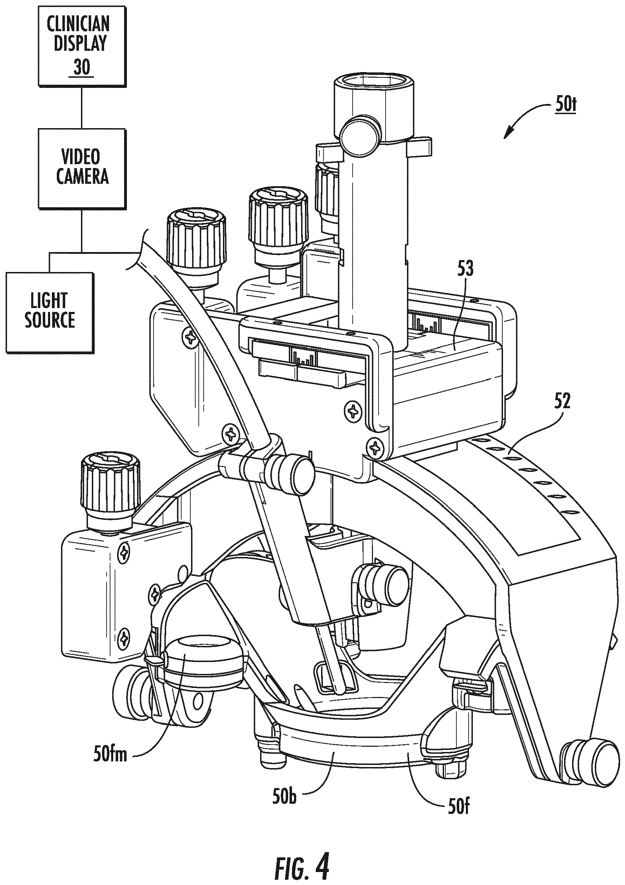

An exemplary trajectory guide **50t** is illustrated in **FIGS. 1-3** in position on a patient. As shown, the trajectory guide **50t (****FIG. 4****)** includes a guide frame **50f,** a targeting cannula **60** and trajectory guide actuators **51** having respective actuator cables **50a (****FIG. 2****)** (providing X-Y adjustment and pitch and roll adjustment) in communication with a trajectory adjustment controller **57.** The frame **50f** can include a control arc **52** that cooperates with a platform **53** to provide pitch and roll adjustments. The platform **53** can allow for X-Y adjustments of the trajectory. The trajectory guide **50t** may include a plurality of MRI-visible frame fiducial markers **50fm** around a base **50b** thereof. For additional discussion of suitable trajectory guides, *see,* U.S. Published Patent Application No. 2009/0112084 (Attorney Docket No. 9450-34IP).

As shown in **FIG. 5****,** the targeting cannula **60** includes an open center lumen or through passage **61** along the axis of the targeting cannula **60.** The distal end portion of the targeting cannula **60** can include a fiducial marker **60m** (typically including a fluid-filled component **65**), shown as a substantially spherical or round (cross-section) marker shape. The proximal end **60p** can be configured with a fluid filled channel **68** concentric with the passage **61** that can define a cylindrical fiducial marker. Other fiducial marker types can be used. The cannula **100** can be slidably introduced and/or withdrawn through the lumen or passage **61.**

An MRI scanner interface **40 (****FIG. 1****)** may be used to allow communication between the workstation **30** and the scanner **20.** The interface **40** and/or circuit **30c** may be hardware, software or a combination of same. The interface **40** and/or circuit **30c** may reside partially or totally in the scanner **20,** partially or totally in the workstation **30,** or partially or totally in a discrete device therebetween.

The MRI scanner **20** can include a console that has a "launch" application or portal for allowing communication to the circuit **30c** of the workstation **30.** The scanner console can acquire volumetric T1-weighted (post-contrast scan) data or other image data (*e.g.,* high resolution image data for a specific volume) of a patient's head or other anatomy. In some embodiments, the console can push DICOM images or other suitable image data to the workstation **30** and/or circuit **30c.** The workstation **30** and/or circuit **30c** can be configured to passively wait for data to be sent from the MR scanner **20** and the circuit **30c**/workstation **30** does not query the scanner or initiate a communication to the scanner. In other embodiments, a dynamic or active communication protocol between the circuit **30c**/workstation **30** and the scanner **20** may be used to acquire image data and initiate or request particular scans and/or scan volumes. Also, in some embodiments, pre-DICOM, but reconstructed image data, can be sent to the circuit **30c**/workstation **30** for processing or display. In other embodiments, pre-reconstruction image data (*e.g*., substantially "raw" image data) can be sent to the circuit **30c**/workstation **30** for Fourier Transform and reconstruction.

Generally described, for some unilateral scenarios, the user (*e.g*., doctor or surgeon) will proceed through a set of discrete workflow steps to load MR image data, identify a target point, identify an entry point, verify the planned trajectory, and align the targeting cannula **60.** A target point or region can also be planned or refined based on real-time functional image data of a patient. The functional image data can include, but is not limited to, images of fiber tracks, images of activity in brain regions during vocalization (*e.g*., reading, singing, talking), or based on physical or olfactory or sense-based stimulation, such as exposure to electrical (discomfort/shock input), heat and/or cold, light or dark, visual images, pictures or movies, chemicals, scents, taste, and sounds or the like) and/or using fMRI or other imaging techniques. The enhanced visualization may give neurosurgeons a much clearer picture of the spatial relationship of a patient's brain structures. The visualizations can serve as a trajectory guide for delivering a substance to the body (*e.g*., to the brain) via the surgical (intrabody) delivery cannula **100.** In some embodiments, the visualizations can be generated using data collated from different types of brain-imaging methods, including conventional magnetic resonance imaging (MRI), functional MRI (fMRI), diffusion-tensor imaging (DTI) and even hyperpolarized noble gas MRI imaging. The MRI gives details on the anatomy, fMRI or other active stimulation-based imaging protocol can provide information on the activated areas of the brain, and DTI provides images of the network of nerve fibers connecting different brain areas. The fusion of one or all of these different images and the tool information can be used to produce a 3-D display with trajectory information that surgeons can manipulate.

Thus, a target location and trajectory can be planned, confirmed or refined based in-part on functional information of the patient. This functional information can be provided, in a user interface (UI) displayed on the display screen **32,** in near real-time visualizations of the patient with the trajectory guide tools for trajectory path and target planning, *e.g*., visualize a patient's fiber track structures and/or functional information of a patient's brain for a surgeon's ease of reference. Knowing where susceptible or sensitive brain regions are or where critical fiber tracks are in the patient's brain, can allow a surgeon to plan a better or less-intrusive trajectory and/or allow a surgeon to more precisely reach a desired target site and/or more precisely place a device and/or deliver a planned therapy substance.

To align the targeting cannula **60,** scan volumes can be defined by the system based on known dimensions of the cannula, such as a cannula length, a position of a proximal or distal marker on the cannula, and angulation and lateral (X-Y) pivot limit.

An estimated distance from the distal tip of the cannula **100** to a reference point on the guide frame **50t (****FIGS. 2** and **3****)** or the targeting cannula **60** (*e.g*., the proximal end of the targeting cannula **60**) can be determined and physically or visually marked on the cannula **100.** The depth stop **70** can be secured about the cannula **100** at the marked location. The depth stop **70** can serve to limit the depth of insertion of the cannula **100** into the patient in a subsequent insertion step or steps.

The user can then (gradually) advance the cannula **100** and acquire images (on the display of the UI) to verify the trajectory and/or avoid functionally sensitive structure as appropriate. When the delivery cannula **100** has been advanced to the target point, high-resolution confirmation images can be obtained to verify the cannula tip location relative to the planned location. Additionally or alternatively, electrical activity can be sensed using an electrode at a tip of the cannula **100.** If actual placement is not correct, the cannula **100** can be withdrawn. At that point, either the X-Y placement can be adjusted appropriately (*e.g*., by moving a platform or stage an amount to cause the desired adjustment) or a trajectory angulation can be re-planned and a second attempt can be made.

For some bilateral scenarios, the above steps can be repeated for both left and right sides, with the additional goal that the patient should not be moved into or out of the scanner. To satisfy that goal, trajectory planning should be completed for both sides prior to removing the patient from the scanner. Also, burring and frame attachment (the member that holds the trajectory guide to the patient's head) should be completed for both sides prior to moving the patient back into the scanner **20** to promote speed of the procedure.

The system **10** can be configured with a (hardware/software) interface that provides a network connection, *e.g.,* a standard TCP/IP over Ethernet network connection, to provide access to MR scanner **20,** such as the DICOM server. The workstation **30** can provide a DICOM C-STORE storage class provider. The scanner console can be configured to be able to push images to the workstation **30** and the workstation **30** can be configured to directly or indirectly receive DICOM MR image data pushed from an MR scanner console. Alternatively, as noted above, the system can be configured with an interface that allows for a dynamic and interactive communication with the scanner **20** and can obtain image data in other formats and stages (*e.g*., pre-DICOM reconstructed or raw image data).

As noted above, the system **10** is configured so that hardware, *e.g*., the trajectory guide 50t and/or the cannula **100,** constitute a point of interface with the system (software or computer programs) because the circuit **30c** is configured with predefined tool data that recognizes physical characteristics of specific tool hardware.

The system **10** may also include and implement a marking grid and/or non-uniformly spaced-apart frame fiducial markers as disclosed in U.S. Patent Application Serial No. 12/236,854, published as U.S. Published Patent Application No. 2009/0171184.

In some embodiments, circuit **30c** can be configured so that the program application can have distinct ordered workflow steps that are organized into logical groups based on major divisions in the clinical workflow as shown in **Table 2.** A user may return to previous workflow steps if desired. Subsequent workflow steps may be non-interactive if requisite steps have not been completed. The major workflow groups and steps can include the following features or steps in the general workflow steps of "start", "plan entry", "plan target", "navigate", and "refine," ultimately leading to delivering and visualizing the therapy (*i.e*., delivering the substance to the target through the cannula **100**) as described in **Table 2.**

**Table 2: Exemplary Clinical Workflow Groups/Steps**

| **Group** | **Step** | **Description** |
|---|---|---|
| Start | Start | Set overall procedure parameters (Optionally confirm hardware compatibility) |
| Plan Entry | ACPC | Acquire a volume and determine AC, PC, and MSP points |
| | Target | Define initial target point(s) for entry planning |
| | Trajectory | Explore potential trajectories to determine entry point(s) |
| | Grid | Locate physical entry point via fiducial grid. |
| Plan Target | ACPC | With hole burred and frame attached, acquire a volume and determine revised AC, PC, and MSP points. |
| | Target | Acquire high-resolution slabs (*e.g*., T2 slabs) to determine target positions in new volume. |
| | Trajectory | Review final planned trajectory prior to starting procedure. |
| Navigate | Initiate | Acquire slabs to locate initial position of cannula. |
| | Alignment | Dynamically re-acquire scan showing position of top of cannula. With each update show projected target position to determine when alignment is correct. |
| | Insertion | Acquire slabs as cannula **100** is inserted into brain. Verify that cannula **100** is following planned trajectory. |
| Refine | Target | Acquire images with cannula **100** in place. Review position and redefine target if necessary. |
| | Adjust XY Offset | Dynamically re-acquire scan showing position of bottom of targeting cannula **60.** With each update show projected target position to determine when offset is correct. |
| | Insertion | Acquire slabs as cannula **100** is inserted into brain. Verify that cannula **100** is following planned trajectory. |
| Delivery or Withdrawal | Substance Delivery | Once cannula **100** position is finalized, prompt user to begin delivery or withdrawal of substance through cannula **100.** |
| | Watch Diffusion Delivery Pattern | Acquire slabs as substance is delivered into or withdrawn from brain and display. |
| Admin | Admin | Reporting and Archive functionality |

The AC, PC and MSP locations can be identified in any suitable manner. In some embodiments, the AC-PC step can have an automatic, electronic AC, PC MSP Identification Library. The AC, PC and MSP anatomical landmarks define an AC-PC coordinate system, *e.g*., a Talairach-Tournoux coordination system that can be useful for surgical planning. This library can be used to automatically identify the location of the landmarks. It can be provided as a dynamic linked library that a host application can interface through a set of Application Programming Interface (API) on Microsoft Windows^{®}. This library can receive a stack of MR brain images and fully automatically locates the AC, PC and MSP. The success rate and accuracy can be optimized, and typically it takes a few seconds for the processing. The output is returned as 3D coordinates for AC and PC, and a third point that defines the MSP. This library is purely computation and is typically UI-less. This library can fit a known brain atlas to the MR brain dataset. The utility can be available in form of a dynamic linked library that a host application can interface through a set of Application Programming Interface (API) on Microsoft Windows ^{®}. The input to this library can contain the MR brain dataset and can communicate with applications or other servers that include a brain atlas or include a brain atlas (*e.g*., have an integrated brain atlas). The design can be independent of any particular atlas; but one suitable atlas is the Cerefy^{®} atlas of brain anatomy (note: typically not included in the library). The library can be configured to perform segmentation of the brain and identify certain landmarks. The atlas can then be fitted in 3D to the dataset based on piecewise affine transformation. The output can be a list of vertices of the interested structures.

In some embodiments, the mid-sagittal plane (MSP) is approximated using several extracted axial slices from the lower part of the input volume, *e.g.,* about 15 equally spaced slices. A brightness equalization can be applied to each slice and an edge mask from each slice can be created using a Canny algorithm. A symmetry axis can be found for each edge mask and identify the actual symmetry axis based on an iterative review and ranking or scoring of tentative symmetry axes. The ranking/scoring cam be based on whether a point on the Canny mask, reflected through the symmetry axis lands on the Canny mask (if so, this axes is scored for that slice). An active appearance model (AAM) can be applied to a brain stem in a reformatted input stack with the defined MSP to identify the AC and PC points.

The MSP plane estimate can be refined as well as the AC and PC points. The MSP plane estimate can be refined using a cropped image with a small region that surrounds a portion of the brain ventricle and an edge mask using a Canny algorithm. The symmetry axis on this edge mask if found following the procedure described above. The AC and PC points are estimated as noted above using the refined MSP and brightness peaks in a small region (*e.g*., 6x6 mm) around the estimate are searched. The largest peak is the AC point. The PC point can be refined using the PC estimate above and the refined MSP. A Canny edge map of the MSP image can be computed. Again, a small region (*e.g*., about 6 mm x 6mm) can be searched for a point that lies on a Canny edge and for which the image gradient is most nearly parallel to the AC-PC direction. The point is moved about 1mm along the AC-PC direction, towards PC. The largest intensity peak in the direction perpendicular to AC-PC is taken to be the PC point.

It will be appreciated that when the target is a tumor or ventricle to be infused or the like, the AC-PC points typically will not be used to provide guidance.

The Navigation-Insertion step may include further aspects as described in **Table 3A:**

**Table 3A: Navigate - Insertion**

| **Description** |
|---|
| The application can provide a depth value to set on the cannula **100** prior to insertion. |
| The application can prompt with scan parameters for oblique coronal and sagittal planes aligned to the trajectory. Also for an oblique axial perpendicular to the trajectory. |
| On receiving coronal or sagittal images, the application can display an overlay graphic indicating the planned trajectory. The most recent coronal and sagittal images can appear together in a 1x2 display. |
| On receiving a trajectory axial scan perpendicular to the trajectory, the application can segment out the cross-sections of the cannula **100** to determine the actual path being followed by the cannula **100.** |
| On receiving a trajectory axial scan perpendicular to the trajectory, the application can display two viewports containing: |
| the axial stack with graphic overlays showing the detected path of the cannula **100** on each image |
| an anatomic axial view through the target showing the planned target and the target projected from the detected path of the cannula **100.** An error value can show the distance between the current projected target and the planned target. |
| If multiple trajectories have been defined for a single entry, the application can display the trajectory that is currently aligned during insertion. |

The application may provide a depth value that is the expected distance from the target to the top of the targeting cannula **60.** The operator can measure the depth value distance from the distal tip of the cannula **100** and mark the proximal end point on the cannula **100** (*e.g*., with a sterile marker). The depth stop **70** can then be secured at the marked location and the measured insertion distance verified. The depth stop **70** is configured to limit a distance that the cannula **100** extends into the body of a patient when the depth stop is inserted within the targeting cannula **60,** so that full insertion of the cannula **100** up to the depth stop will provide the desired insertion depth.

In the event that the placement is not acceptable, the user may opt to proceed to the X-Y Adjustment workflow step as described in **Table 3B:**

**Table 3B: Refine - Adjust X- Y Offset**

| **Description** |
|---|
| The X-Y Adjustment step can display the current target and projected point as annotations to the image data that was acquired during the Target Refinement step. |
| This step can prompt the user to acquire 2D images with scan plane parameters such that the image lies perpendicular to the trajectory and through the pivot point. |
| On receiving a 2D image through the pivot point, the step can calculate the current projected target. |
| This step can display lines from the current projected target to the revised target that indicate the track the projected target would travel if the X and Y offset wheels were turned independently. The lines can be colored to match colors on the control wheels for X and Y offset respectively. A tool-tip (*e.g*., pop-up) can provide text to describe the necessary action. |
| *(For example: "Turn X-offset knob to the Left")* |
| This step can display an annotation indicating the location of the original planned target. |
| When drawing the target and the current projection of the cannula path, the annotations can be drawn to match the physical size of the cannula **100** diameter. |

After the cannula **100** has been placed and the position has been accepted by the user, the user may proceed to the substance delivery or withdrawal step.

Again, it is noted that functional patient data can be obtained in near real-time and provided to the circuit **30c**/workstation **30** on the display **32** with the visualizations of the patient anatomy to help in refining or planning a trajectory and/or target location for placement of the cannula **100.**

The system **10** can provide a UI to set target points so that the trajectories through potential entry points can be investigated. The user may opt to overlay the outlines from a standard brain atlas over the patient anatomy for comparison purposes which may be provided in color with different colors for different structures. When using the brain atlas, the user may opt to show either just the target structure (STN or GPi) or all structures. In either case, a tooltip (*e.g*., pop-up) can help the user to identify unfamiliar structures. The user may also opt to scale and/or shift the brain atlas relative to the patient image to make a better match. To do this, the user may drag the white outline surrounding the brain atlas template. Fiber track structures and/or functional information of a patient's brain can be provided in a visually prominent manner (*e.g*., color coded or other visual presentation) for a surgeon's ease of reference.

The UI can display images and information that enable the user to see how well the cannula **100** is following the planned trajectory. The user may opt to scan Axial, Coronal and Sagittal slabs along the cannula **100** to visually determine the cannula **100** alignment in those planes. The user can also scan perpendicular to the cannula **100.** In that case, the circuit **30c** (*e.g.*, software) can automatically identify where the cannula **100** is in the slab and it then shows a projection of the current path onto the target plane to indicate the degree and direction of error if the current path is continued. The user can perform these scans multiple times during the insertion. The automatic segmentation of the cannula **100** and the display of the projected target on the target plane provide fully-automatic support for verifying the current path. The Coronal/Sagittal views can provide the physician with a visual confirmation of the cannula **100** path that does not depend on software segmentation.

After completing the initial insertion of the cannula **100,** the user (*e.g*., physician) may find that either the placement does not correspond sufficiently close or perfectly to the plan, or the plan was not correct. The UI can support functionality whereby the physician can withdraw the cannula **100** and use the X and Y offset adjustments to obtain a parallel trajectory to a revised target. The UI can prompt the user or otherwise acquire an image slab through the distal tip of the cannula **100.** The UI can display the slab and on it the user may opt to modify the target point to a new location or accept the current position as final.

The UI can also support the user in adjusting a small X-Y offset to set the targeting cannula **60** to a trajectory parallel to the original one. The UI can provide visualization of the position of the cannula **100** tip relative to the target and with instructions on what physical adjustments to make to obtain the desired parallel trajectory (shown as "turn Y wheel to the right") and the projected error.

After the angular and/or X-Y adjustments are made, the cannula **100** insertion is carried out in the same manner as described above.

After the cannula **100** has been inserted and had its position verified by the physician, the UI can prompt the physician to begin delivery of the substance to the target via the cannula **100.** In some embodiments, a test spray of a biocompatible fluid of similar density to the target therapeutic substance (*e.g*., saline) may be first delivered to the target.

The physician (or other operator) then actuates the pump (or syringe) **82** to begin driving a flow of the therapeutic substance through the tubing **84** and the lumen **112** of the cannula **100.** A mass flow of the substance exits the cannula **100** through the exit port **116** into the target region **T** or the vicinity of the target region **T.**

Using MRI image data, the system **10** may render or generate near real time visualizations of the infused or delivered substance along with the near real time visualizations of the target anatomical space and the cannula **100** in the UI. That is, in the same or similar manner to the segmentation and visualization/display of the patient anatomy, the application can segment out the cross-sections of the delivered substance to determine the actual volume occupied by the delivered substance. Scans of scan planes proximate the distal tip of the cannula **100** or associated with target regions can be acquired. The MR image data can be obtained and the actual distribution of the delivered substance in tissue can be shown on the display. These visualizations can be dynamically rendered (*e.g*., in near real time) to show the dynamic dispersion and/or infusion pattern and/or path of the delivered substance. In some embodiments, an MR contrast agent or fluid can be provided in the delivered substance having an increased SNR relative to the tissue.

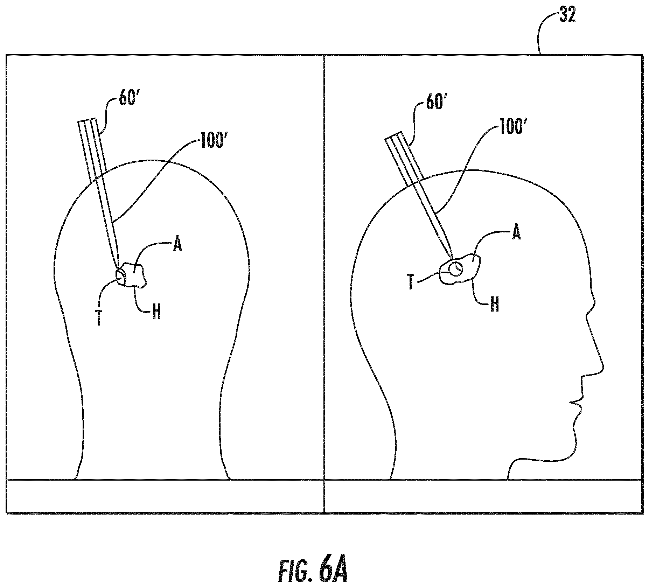

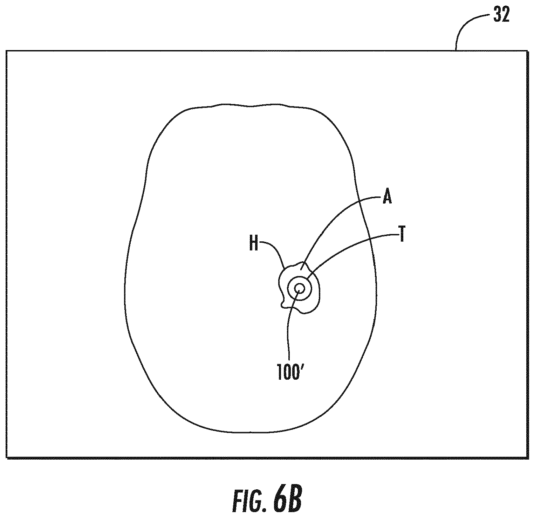

**FIG. 6A** illustrates a screen shot from the UI that allows the user to see the distribution of the delivered substance, which is represented in the UI by the displayed image **A.** This screen shot illustrates coronal and sagittal views to the target (*e.g*., STN). The user may opt to scan Coronal and Sagittal slabs along the cannula **100** to visually determine the distribution pattern or flow paths in those planes. The user can also scan perpendicular to the cannula **100.** The cannula **100** is represented in the screen shot by an image **100'** and the targeting cannula **60** is represented in the screen shot by an image **60'.** **FIG. 6B** illustrates an axial slab through the target **T.** The user can perform these scans multiple times during delivery of the substance. The automatic segmentation and display of the delivered substance, the patient anatomy and the cannula **100** provide fully-automatic support for verifying or assessing the anatomical flow paths, diffusion, dispersion, permeation and/or other distribution of the delivered substance in the patient.

The delivered substance may be visually highlighted in the display of the UI. For example, a graphical overlay or outline **H** may be provided in one or more of the displayed views that highlights the image of the delivered substance **A.** By way of further example, the image of the delivered substance **A** in the tissue may be provided with a contrasting coloring or shading.

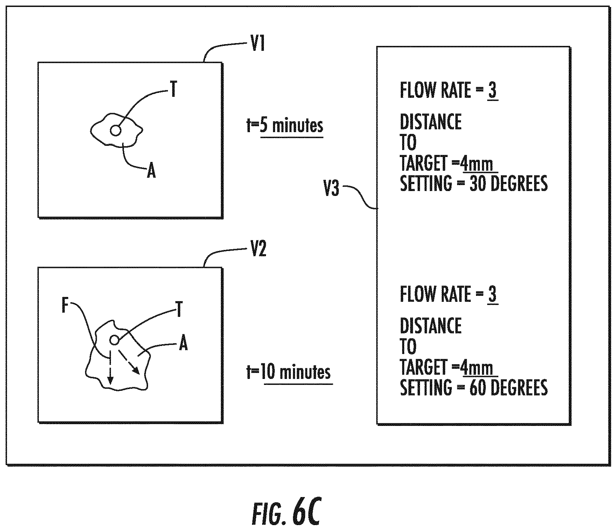

As noted above, the operator can perform scans multiple times during the procedure to track or assess the delivery performance. The UI may allow the operator to display the MR image data in a manner that assists the operator in comparing the flow and/or distribution of the delivered substance over time. **FIG. 6C** illustrates a screen shot from the UI including a view **V1** including an image **A** of the delivered substance at a first time (*e.g*., "t = 5 minutes") and a view **V2** including an image **A** of the delivered substance **A** at a second, subsequent time (*e.g*., "t =10 minutes"). The UI may also display (*e.g*., in a view **V3**) relevant data such as the pump settings, flow rate, delivery cannula port settings, and/or nozzle angle at each of the selected times or any intervening adjustments. The UI may also display indicia **F,** such as graphical arrows indicating the directions of flow or dispersion, for example, between the selected times or over time. The foregoing visualization aids may assist the operator in assessing the effect of adjustments in flow rates, delivery cannula placement, or delivery cannula settings, for example.

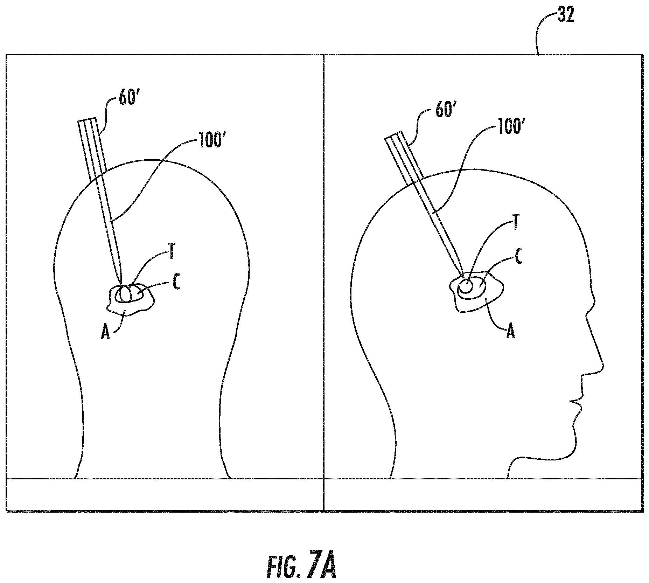

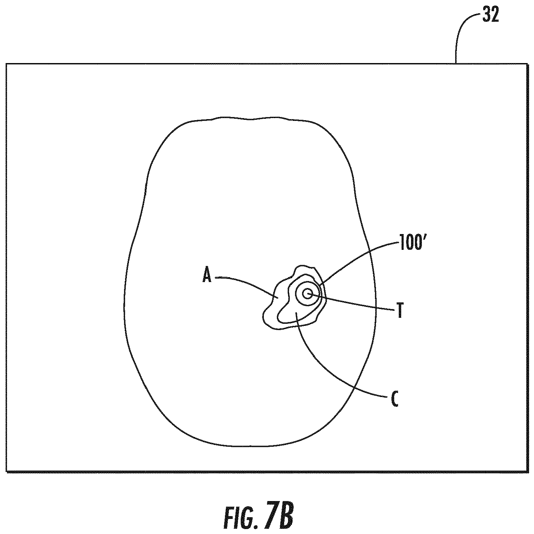

**FIG. 7A** illustrates a screen shot from the UI in the case where the target region is a tumor site in a patient's brain. The tumor site is segmented and displayed as a part of the patient anatomy and the image **C** of the tumor may be highlighted, enhanced, contrasted or otherwise augmented to enable the operator to more easily discern the tumor image **C** in the displayed image. The UI enables the user to see the distribution of the delivered or infused substance with respect to the tumor site. This screen shot illustrates coronal and sagittal views through the tumor. The user may opt to scan Coronal and Sagittal slabs along the cannula **100** to visually determine the distribution pattern or flow paths in those planes. The user can also scan perpendicular to the cannula **100.** **FIG. 7B** illustrates an axial slab through the tumor. The user can perform these scans multiple times during delivery of the substance. The automatic segmentation and display of the delivered substance, the patient anatomy (including the tumor) and the cannula **100** provide fully-automatic support for verifying or assessing the anatomical flow paths, diffusion, dispersion and distribution of the delivered substance with respect to the tumor site and the remainder of the patient anatomy.

The operator can use the feedback from the UI to assess, re-plan and/or modify the infusion or delivery procedure.

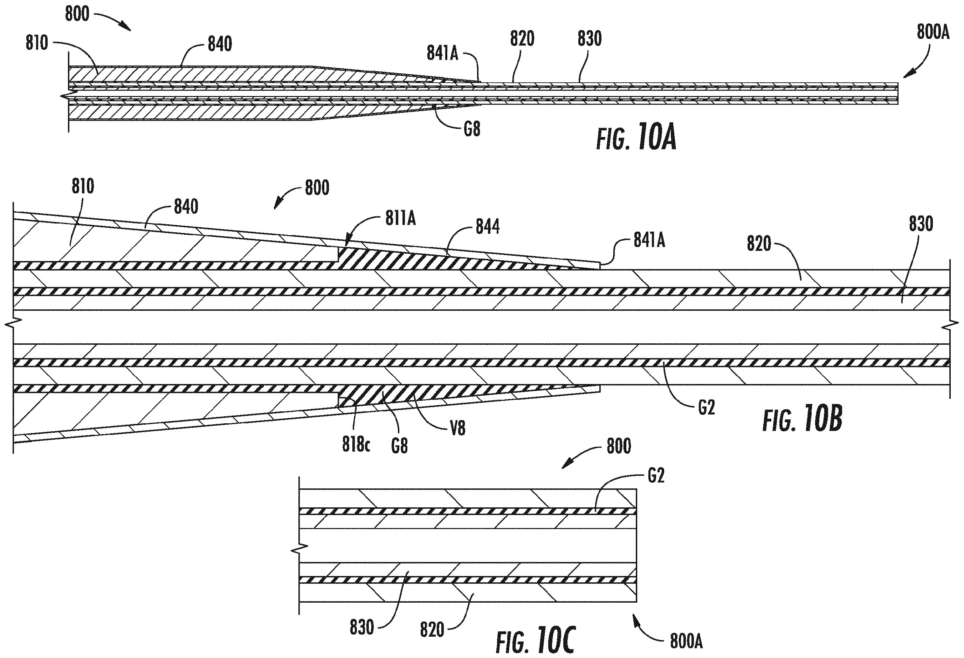

Responsive to the UI feedback, the operator may adjust one or more operational parameters during the fluid delivery. For example, the mass flow rate of the substance exiting the cannula **100** can be increased or decreased. This may be accomplished by adjusting the mass flow rate setting of the pump **82** or a regulator (*e.g*., restrictor or valve) upstream of the cannula **100.** In some cases (*e.g*., as described below with reference to **FIG. 10**)**,** the delivery cannula includes a flow control mechanism that can be used to adjust the mass flow rate of delivery.

The operator may adjust the placement of the exit port responsive to the UI feedback. For example, the operator may insert the cannula **100** further into the patient or withdraw the end of the cannula **100** somewhat from the patient. The operator may fully withdraw the cannula **100** from the patient, plan a new target and trajectory, re-insert the cannula **100** to the new target, and re-initiate delivery of the substance to the new target through the cannula **100.** This procedure may be used one or more times in order to dispense the substance into different regions of a tumor, for example. Different cannulas or a protective (*e.g*., retractable) sheath may be used to inhibit spread of tumor cells.

With reference to **FIGS. 8A-8F****,** a cannula system **601** according to embodiments of the present disclosure is shown therein. The system **601** includes an intrabody surgical (*e.g*., delivery) cannula **600** (hereinafter, the delivery cannula **600**), which can optionally be MRI compatible. The cannula **600** can be coupled to connecting tubing **660,** and a luer fitting (*e.g*., luer lock) **652.** The cannula **600** may be used in place of the cannula **100** and the connecting tubing **660** may be used in place of the connecting tubing **84.** The cannula **600** may be particularly well-suited for delivering a substance into a patient.

The cannula **600** includes a rigid tubular support sleeve **610,** an inner sleeve **620,** a transfer tube **630,** an outer conformal polymeric sleeve **640,** and adhesives **G1** and **G2.** The cannula **600** extends from a proximal end **600B** to a distal end **600A.**

The rigid tubular support sleeve **610** defines an axially extending central lumen **612.** A first or inlet opening **616** on the proximal end of the support sleeve **610** and a second or exit opening **614** on the distal end **611A** of the support sleeve **610** each fluidly communicate with the lumen **612.** The outer surface **618** of the support sleeve **610** includes a proximal section **618A** having a substantially uniform diameter **D8.** The outer surface **618** also includes a distal section **618B** having a tapered or frusto-conical shape that tapers in the axial distal direction. The outer surface **618** further includes a distal end face **618C** at the distal end **611A.**

According to some embodiments, the support sleeve **610** is formed of a substantially rigid MRI-compatible material. According to some embodiments, the support sleeve **610** is formed of an MR safe material. According to some embodiments, the MRI-compatible material is a ceramic. Suitable ceramics may include Alumina. Other MRI-compatible materials that may be used for the sleeve **610** may include glass or rigid polymers.

The inner sleeve **620** extends through the lumen **612** to a distal end **621A** of the inner sleeve. The inner sleeve **620** is secured to the inner surface of the support sleeve **610.** According to some embodiments, the inner sleeve **620** is bonded to the inner surface of the support sleeve **610** by a layer of adhesive **G1** such as LOCTITE^{®} 4014 adhesive.

The inner sleeve **620** defines an axially extending central lumen **622.** A first or inlet opening **626** on the proximal end of the inner sleeve **620** and a second or exit opening **624** on the distal end **621A** of the inner sleeve **620** each fluidly communicate with the lumen **622.** An extension section **620A** of the inner sleeve **620** extends beyond the distal end of the support sleeve **610** and is exposed. The distal end **621A** of the inner sleeve **620** defines a distal end face **620B.** A proximal extension section **620C** of the inner sleeve **620** extends beyond the proximal end of the support sleeve **610** and through the connecting tubing **660** to a delivery pump, for example.

According to some embodiments, the inner sleeve **620** is formed of a substantially rigid MRI-compatible material. According to some embodiments, the MRI-compatible material is fused silica.

A transfer tube **630** extends through the lumen **622** to a distal end **631A** of the transfer tube **630.** The transfer tube **630** is secured to the inner surface of the inner sleeve **620.** According to some embodiments, the transfer tube **630** is bonded to the inner surface of the inner sleeve **620** by a layer of adhesive **G2,** such as LOCTITE^{®} 4014 adhesive.

The transfer tube **630** defines an axially extending central lumen **632.** A first or inlet opening **636** on the proximal end of the transfer tube **630** and a second or exit opening **634** on the distal end **631A** of the transfer tube **630** each fluidly communicate with the lumen **632.** A distal extension section **630A** of the transfer tube **630** extends beyond the distal end **620A** of the inner sleeve **620** and is exposed. A proximal extension section **630C (****FIG. 8C****)** of the transfer tube **630** extends beyond the proximal end of the support sleeve **610** and through the connecting tubing **660** to a delivery pump, for example.

According to some embodiments, the transfer tube **630** is formed of a substantially rigid MRI-compatible material. According to some embodiments, the MRI-compatible material is fused silica.

The outer conformal outer polymeric sleeve **640** surrounds and fits tightly about the support sleeve **610.** The conformal polymeric sleeve **640** extends from a proximal end proximate the connecting tubing **660** to a distal end **641A (****FIG. 8E****)** at the distal end **621A** of the inner sleeve **620.** Thus, the conformal polymeric sleeve **640** is coterminous with the inner sleeve **620** at their distal ends.

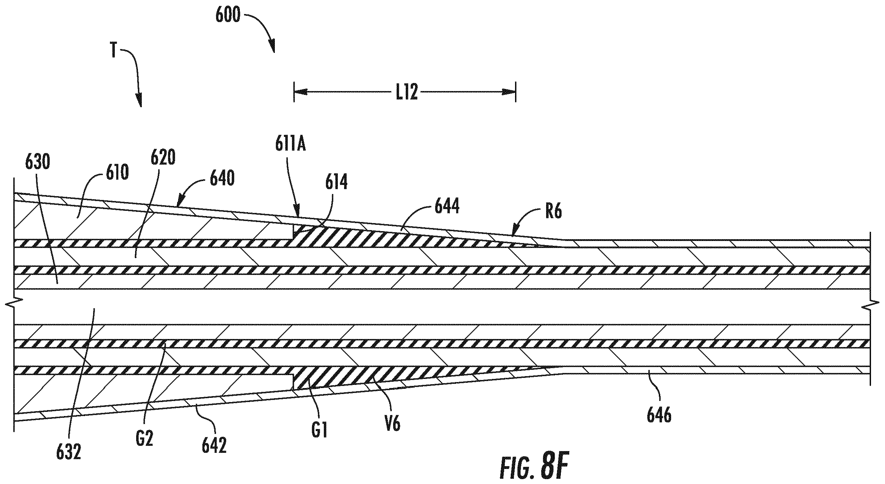

With reference to **FIG. 8F****,** the conformal polymeric sleeve **640** includes a proximal section **642,** a transitional section **644,** and a distal section **646.** The proximal section **642** surrounds a portion of the support sleeve **610.** The distal section **646** surrounds a portion of the inner sleeve **620.** The transitional section **644** begins at and extends from the distal end **611A** of the support sleeve **610** and over the inner sleeve **620** in the direction toward the distal end **621A** of the inner sleeve **620** (*i.e.,* toward the tip of the cannula **600**)**.** The transitional section **644** connects the sections **642** and **646** and surrounds a portion of the inner sleeve **620** adjacent the distal end **611A** of the support sleeve **610.**

The transitional section **644** is frustoconical and tapers inwardly in axial distal direction. In this manner, the transitional section **644** reduces in diameter from the outer diameter of the support sleeve **610** at the end face **614** to the outer diameter of the inner sleeve 620.

An annular void **V6** is defined between the outer surface of the inner sleeve **620** and the inner surface of the transitional section **644.** The outer diameter of the void **V6** is frusto-conical. The void **V6** extends from the distal end **611A** of the support sleeve **610** to the location or region **R6 (****FIG. 8F****)** where the conformal sleeve meets, engages, or conforms to the outer diameter surface of the inner sleeve **620.**

The void **V6** is at least partially filled with the solid, rigid adhesive **G1.** As a result, the transitional section **644** and the adhesive **G1** therein form a solid ramp from the outer diameter of the inner sleeve **620** to the outer diameter of the support sleeve distal end section **618B.** In this way, the step at the support sleeve end face **618C** is effectively smoothed of eliminated on the outer diameter of the cannula **600.** In some embodiments, the void **V6** is fully filled with the solid, rigid adhesive **G1.**

In some embodiments, the length **L12 (****FIG. 8F****)** is in the range of from about 0.1 mm to 5 mm.

The distal section **646** surrounds the remainder of the inner sleeve **620** extending beyond the location **R6.** The distal section **646** extends from the location **R6** to the distal end **641A** of the conformal polymeric sleeve **640.** The distal section **646** is thus coterminous with the inner sleeve **620** at the distal end **621A.** In some embodiments, the polymeric sleeve distal section **646** wraps around the end **621A** of the inner sleeve **620,** extends radially inward to the transfer tube **630,** and covers the inner sleeve end face **620B.**

According to some embodiments, the conformal polymeric sleeve **640** is formed of polyethylene terephthalate (PET). According to some limits, the conformal polymeric sleeve **640** is an elastomeric shrinkable sleeve.

The connecting tubing **660 (****FIGS. 8A-8D****)** is coupled to the proximal end of the support sleeve **610** by a tubing adapter **662.** The tubing adapter **662** may be bonded to the support sleeve **610** and the connecting tubing **660** by an adhesive **G3,** such as LOCTITE^{™} UV 3311 adhesive. The connecting tubing **660** may be formed of any suitable MRI compatible material. According to some embodiments, the connecting tubing **660** is formed of polyvinyl chloride (PVC). According to some embodiments, the connecting tubing **660** is formed of silicone. The tubing adapter **662** may be formed of any suitable MRI compatible material, such as an MRI-compatible polymer.

The luer fitting **652** is coupled to the proximal end of the connecting tubing **660** by a luer adapter **654.** The luer adapter **654** may be bonded to the luer fitting **652** and the connecting tubing **660** by an adhesive **G4,** such as LOCTITE^{™} UV 3311 adhesive. The luer adapter **654** may be formed of any suitable MRI compatible material, such as an MRI-compatible polymer. The luer fitting **652** may also be bonded to the inner sleeve **620** by one or more the adhesives **G5, G6,** such as LOCTITE^{®} UV 3311 adhesive and/or LOCTITE^{®} UV 4014 adhesive.

According to some embodiments, the inner diameter **D1** of the transfer tube **630** is in the range of from about 10 µm to 1 mm and, in some embodiments, is about 200 µm. According to some embodiments, the outer diameter **D2** of the transfer tube **630** is in the range of from about 75 µm to 1.08 mm and, in some embodiments is about 360 µm. According to some embodiments, the length **L1** of the exposed section **630A** of the transfer tube **630** is in the range of from about 1mm to 50 mm and, in some embodiments is about 3mm.

According to some embodiments, the inner diameter **D4** of the inner sleeve **620** is in the range of from about 85 µm to 1.1 mm and, in some embodiments, is about 450 µm. According to some embodiments, the outer diameter **D5** of the inner sleeve **620** is in the range of from about 150 µm to 1.5 mm and, in some embodiments, is about 673 µm. According to some embodiments, the length **L4** of the exposed section **620A** of the inner sleeve **620** (*i.e*., the section of the inner sleeve **620** extending distally beyond the support sleeve end face **618C**) is in the range of from about 1 mm to 75 mm and, in some embodiments is about 15 mm.

According to some embodiments, the inner diameter **D7** of the support sleeve **610** is in the range of from about 160 µm to 1.55 mm and, in some embodiments, is about 750 µm. According to some embodiments, the outer diameter **D8** of the uniform diameter section **618A** of the support sleeve **610** is in the range of from about 500 µm to 4 mm and, in some embodiments, is about 1.6 mm. According to some embodiments, the overall length **L7** of the support sleeve **610** is in the range of from about 0.5 inch to 20 inches (1.27 cm to 50.8 cm) and, in some embodiments, is in the range of from about 10 to 14 inches (25.4 cm to 35.56 cm). According to some embodiments, the length **L8** of the tapered section **618B** of the support sleeve **610** is in the range of from about 6 to 9 mm.

According to some embodiments, the thickness of the conformal polymeric sleeve 640 is in the range of from about 40 to 60 µm.

As best seen in **FIG. 8E****,** the cannula **600** is a stepped cannula with three co-axially disposed step segments (the outer surfaces of the transfer tube **630,** the inner sleeve **620** and the conformal polymeric sleeve **640,** respectively) having different outer diameters and separated by the steps or rises. The step formed by the end face **620B** is a sharp step. The frustoconical sections **644, 618B** form a transitional section **T** of the cannula **600** having a smooth rise (*i.e*., not a sharp step).

The cannula **600** may be a unitary, integral structure having no relatively slidably elements.

The cannula **600** may be used in the same manner as described herein with respect to the cannula **100,** for example. The luer can be operatively coupled to an infusion pump (*e.g.,* the infusion pump **82**) or syringe, which supplies a mass flow of the desired substance or material to be delivered into the patient.

The cannula **600** can provide a number of advantages. The rigid support sleeve 610 prevents or inhibits bending or flex of the large majority of the length of the cannula **600** as the cannula **600** is inserted through the targeting cannula **60** and into the patient (*e.g*., the brain). By restricting the axial movement of the cannula **600** during insertion, the cannula **600** can reduce or prevent small movements that may disrupt tissue and thereby lead to reflux of the dispensed or infused substance. A ceramic support sleeve **610,** in particular, can provide good rigidity while also being MRI-compatible and MRI safe. According to some embodiments, the entirety of the cannula **600** is formed of an MRI-compatible, MR safe material or materials.

The conformal polymeric sleeve **640** may beneficially provide a lubricious surface over the support sleeve **610** to reduce shear force on the brain or other tissue during insertion. The conformal polymeric sleeve **640** can enhance the safety of the cannula **600** by capturing the support sleeve **610** or pieces thereof if the support sleeve is accidentally broken *in situ.*

The step at the end face **620B** can serve to reduce or prevent reflux of the delivered substance. The provision of an exposed transfer tube section **630A** having the aforedescribed length **L1** and inner diameter **D1** has also been found to provide beneficial reflux resistance performance.

The tapered transition between the outer diameter **D5** of the inner sleeve **620** and the outer diameter **D7** of the inner sleeve **620** can provide the reflux control of the small diameter inner sleeve **620** along with a support sleeve **610** having a geometry providing satisfactory rigidity and size for cooperation with the targeting cannula **60** or an adapter.

The protective connecting tubing **660** can serve to protect the transfer tube **630** while also permitting convenient routing the connecting tubing **660** to the delivery pump. According to some embodiments, the length of the tubing **660** is in the range of from about 1.83 to 3.66 meters (6 to 12 feet).

According to some embodiments, the delivered substance is delivered to a patient's brain through the exit opening **634** at a delivery rate in the range of from about 1 to 3 µL/minute.

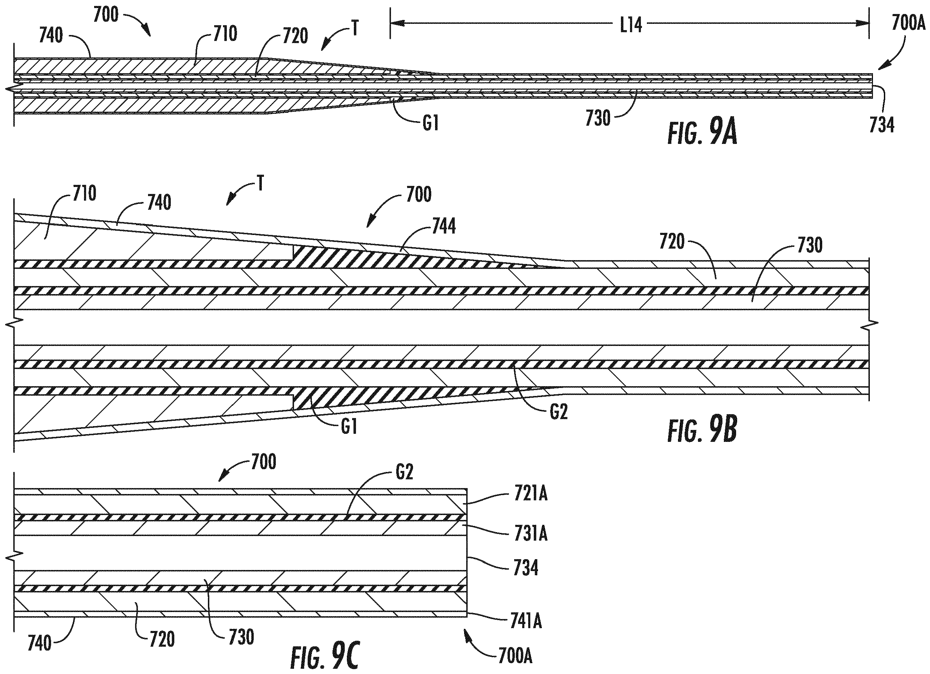

With reference to **FIGS. 9A-9C****,** a cannula **700** according to further embodiments is shown therein. The cannula **700** is constructed in the same manner as the cannula **600,** except as discussed below. The cannula **700** can be used in place of and in the same manner described for the cannula **600** (*e.g.,* with the connecting tubing **660,** and a luer fitting (*e.g.,* luer lock) **652**)**.** The cannula **700** may be particularly well-suited for delivering a substance to a patient.

The cannula **700** includes a rigid tubular support sleeve **710,** an inner sleeve **720,** a transfer tube **730,** a conformal polymeric sleeve **740,** an adhesive **G1,** and an adhesive **G2** constructed and assembled in the same manner as the support sleeve **610,** the inner sleeve **620,** the transfer tube **630,** the conformal polymeric sleeve **640,** the adhesive **G1,** and the adhesive **G2,** respectively, of the cannula **600** except as discussed below. The cannula **700** extends from a proximal end to a distal end **700A.** The cannula **700** includes a conformal sleeve transitional section **744** and a transitional section **T** corresponding to the conformal sleeve transitional section **644** and the transitional section **T** of the cannula **600.**

The cannula **700** differs from the cannula **600** in that the distal end **721A (****FIG. 9C**) of the inner sleeve **720** is substantially coterminous or coincident with the distal end **731A** of the transfer tube **730.**

As a result, the cannula **700** does not include a step corresponding to the step at the distal end **621A** of the inner sleeve **620.** The cannula **700** includes only the smoothly tapered rise at the transitional section **T.** The cannula **700** has a constant outer diameter after the rise at the transitional section **T** to the tip.

In the cannula **700,** the conformal polymeric sleeve **740** terminates at the distal end **721A** of the inner sleeve **620** as described for the cannula **600** (*i.e.,* the conformal sleeve distal end **741A (****FIG. 9C****)** is coterminous with the distal end **721A** and the cannula distal end **700A).**

In some embodiments, the length **L14 (****FIG. 9A****)** of the transfer tube **730** extending beyond the support sleeve **710** is in the range of from about 3 mm to 8 cm.

The cannula **700** may be particularly well-suited for delivering stem cells to the brain tissue of a patient. According to method embodiments of the present disclosure, stem cells are delivered or injected through the cannula **700** into a patient as described herein. In some embodiments, the stem cells are injected into patient brain tissue through the cannula **700.** The stem cells may be suspended in a liquid composition or suspension that is delivered through the cannula **700.**

In some embodiments, the stems cells are delivered to the patient tissue in the following manner. The cannula **700** is inserted into the patient anatomy as described herein to position the distal end **700A** in or proximate a target region. The cannula **700** is then pulled back or withdrawn from the patient a small or prescribed distance such that the distal end **700A** remains in the patient in or proximate the target region, and a gap or channel is thereby formed in the patient tissue where the distal end section of the cannula was formerly located. The stem cells are then dispensed into the patient, including into the formed channel, through the cannula **700** and the distal end opening **734.** In some embodiments, the cannula **700** is then again pulled back or withdrawn from the patient a small or prescribed distance such that the distal end **700A** remains in the patient in or proximate the target region, and a further gap or channel is thereby formed in the patient tissue where the distal end section of the cannula was formerly located. The stem cells are then dispensed into the patient, including into the second formed channel, through the cannula **700** and the distal end opening **734.** This procedure (withdraw the cannula incrementally and inject stem cells) may be repeated any suitable or desired number of times.

In some embodiments, the stem cells are implanted, dispensed or injected into the gap or channel with relatively little or no supplemental pressure applied to the stem cells. Because the stem cells are dispensed into the gap or channel, in some embodiments it is not necessary to pressurize the delivered liquid in order to force the liquid into the patient tissue as desired.

With reference to **FIGS. 10A-10C****,** a cannula **800** according to further embodiments is shown therein. The cannula **800** is constructed in the same manner as the cannula **700,** except as discussed below. The cannula **800** can be used in place of and in the same manner described for the cannula **600** (*e.g*., with the connecting tubing **660,** and a luer fitting (*e.g.,* luer lock) **652**).

The cannula **800** includes a rigid tubular support sleeve **810,** an inner sleeve **820,** a transfer tube **830,** a conformal polymeric sleeve **840,** an adhesive **G8,** and an adhesive **G2** constructed and assembled in the same manner as the support sleeve **710,** the inner sleeve **720,** the transfer tube **730,** the conformal polymeric sleeve **740,** the adhesive **G1,** and the adhesive **G2,** respectively, of the cannula **700,** except as discussed below. The cannula **800** extends from a proximal end to a distal end **800A.**

The cannula **800** differs from the cannula **700** in that the distal end **841A** of the conformal polymeric sleeve **840** is proximate the distal end **811A** of the rigid support sleeve **810** between the distal end **811A** and the distal end **821A** of the inner sleeve **820.** The conformal polymeric sleeve **840** extends over and surrounds the inner sleeve **830.**

The conformal polymeric sleeve **840** includes a transitional section **844** corresponding to the transitional section **644 (****FIG. 8F****)** and that is frustoconical and tapers inwardly in the axial distal direction. The transitional section **844** reduces in diameter from the outer diameter of the support sleeve **810** at the end face **814** to the outer diameter of the inner sleeve **820.**

An annular void **V8** (corresponding to the annular void **V6** of **FIG. 8F**) is defined between the outer surface of the inner sleeve **820** and the inner surface of the transitional section **844.** The void **V8** is at least partially filled with the solid, rigid adhesive **G8.** As a result, the transitional section **844** and the adhesive **G8** therein form a solid ramp from the outer diameter of the inner sleeve **820** to the outer diameter of the support sleeve distal end section. In this way, the step at the support sleeve end face **818C** is effectively smoothed or eliminated on the outer diameter of the cannula **800.** In some embodiments, the void **V8** is fully filled with the solid, rigid adhesive **G8.**

In alternative embodiments (not shown in the drawings), the cannula **800** is constructed such that the distal end **841A** of the conformal polymeric sleeve **840** is located at the distal end **811A** of the rigid support sleeve **810.** In this case, no void **V8** is formed and the adhesive **G8** does not extend beyond the support sleeve **810.** In some embodiments, the conformal polymeric sleeve **840** covers the distal end face of the support sleeve **810** down to the outer diameter of the inner sleeve **820** to smooth the transition from the outer diameter of the support sleeve **810.**

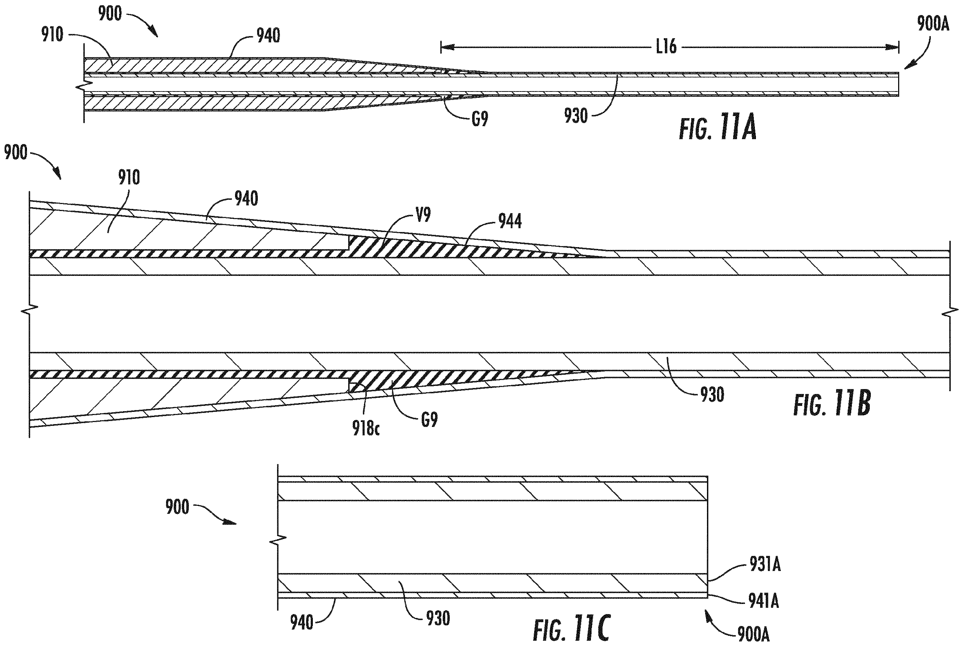

With reference to **FIGS. 11A-11C****,** a cannula **900** according to further embodiments is shown therein. The cannula **900** is constructed in the same manner as the cannula **600,** except as discussed below. The cannula **900** can be used in place of and in the same manner described for the cannula **600** (*e.g.,* with the connecting tubing **660,** and a luer fitting (*e.g*., luer lock) **652**)**.**

The cannula **900** includes a rigid tubular support sleeve **910,** a transfer tube **930,** a conformal polymeric sleeve **940,** and an adhesive **G9** constructed and assembled in the same manner as the support sleeve **610,** the transfer tube **630,** the conformal polymeric sleeve **640,** and the adhesive **G2,** respectively, of the cannula **600** except as discussed below. The cannula **900** extends from a proximal end to a distal end **900A.**

The cannula **900** differs from the cannula **600** in that the cannula **900** does not include a component corresponding to the inner sleeve **620.** Instead, the outer diameter of the transfer tube **930** is bonded directly to the inner diameter of the support sleeve **910** by the adhesive **G2.**

The conformal polymeric sleeve **940** extends over and surrounds the transfer tube **930.** The distal end **941A** of the conformal polymeric sleeve **940** is substantially coterminous or coincident with the distal end **931A** of the transfer tube **930.**

The conformal polymeric sleeve **940** includes a transitional section **944** corresponding to the transitional section **644 (****FIG. 8E****)** and that is frustoconical and tapers inwardly in axial distal direction. The transitional section **944** reduces in diameter from the outer diameter of the support sleeve **910** at the end face **914** to the outer diameter of the transfer tube **930.**

An annular void **V9** (corresponding to the annular void **V6** of **FIG. 8F**) is defined between the outer surface of the transfer tube **930** and the inner surface of the transitional section **944.** The void **V9** is at least partially filled with the solid, rigid adhesive **G9.** As a result, the transitional section **944** and the adhesive **G9** therein form a solid ramp from the outer diameter of the transfer tube **930** to the outer diameter of the support sleeve distal end section **918B.** In this way, the step at the support sleeve end face **918C** is effectively smoothed or eliminated on the outer diameter of the cannula **900.** In some embodiments, the void **V9** is fully filled with the solid, rigid adhesive **G9.**

In some embodiments, the length **L16 (****FIG. 11A****)** of the transfer tube **930** extending beyond the support sleeve **910** is in the range of from about 3 mm to 8 cm.

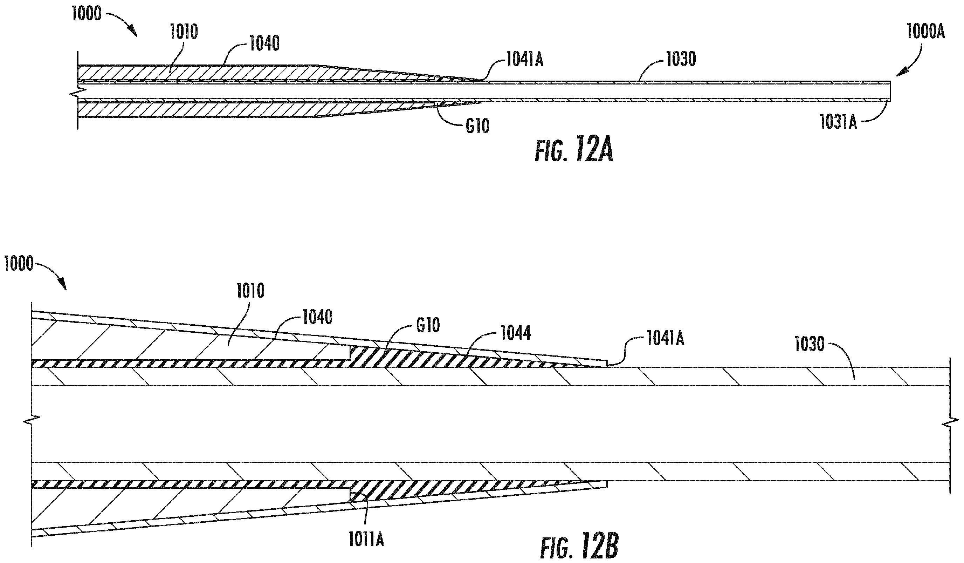

With reference to **FIGS. 12A-12B****,** a cannula **1000** according to further embodiments is shown therein. The cannula **1000** is constructed in the same manner as the cannula **900,** except as discussed below. The cannula **1000** can be used in place of and in the same manner described for the cannula **600** (*e.g.,* with the connecting tubing **660,** and a luer fitting (*e.g.,* luer lock) **652**)**.**

The cannula **1000** includes a rigid tubular support sleeve **1010,** a transfer tube **1030,** a conformal polymeric sleeve **1040,** and an adhesive **G10** constructed and assembled in the same manner as the support sleeve **910,** the transfer tube **930,** the conformal polymeric sleeve **940,** and the adhesive **G9,** respectively, of the cannula **900** except as discussed below. The cannula **1000** extends from a proximal end to a distal end **1000A.** The cannula **1000** includes a conformal sleeve transitional section **1044** and a transitional section **T** corresponding to the conformal sleeve transitional section **944** and the transitional section **T** of the cannula **900.**

The cannula **1000** differs from the cannula **900** in that the distal end **1041A** of the conformal polymeric sleeve **1040** is located at or proximate the distal end **1011A** of the rigid support sleeve **1010.** In some embodiments, the polymeric sleeve **1040** covers the distal end face of the support sleeve **1010** down to the outer diameter of the transfer tube **1030** to smooth the transition from the outer diameter of the support sleeve **1010.**

The cannula **1000** differs from the cannula **900** in that the distal end **1041A** of the conformal polymeric sleeve **1040** is proximate the distal end **1011A** of the rigid support sleeve **1010** between the distal end **1011A** and the distal end **1031A** of the transfer tube **1030.** The conformal polymeric sleeve **1040** extends over and surrounds the transfer tube **1030.**

In alternative embodiments (not shown in the drawings), the cannula **1000** is constructed such that the distal end **1041A** of the conformal polymeric sleeve **1040** is located at the distal end **1011A** of the rigid support sleeve **1010.** In some embodiments, the polymeric sleeve **1040** covers the distal end face of the support sleeve **1010** down to the outer diameter of the transfer tube **1030** to smooth the transition from the outer diameter of the support sleeve 1010.

The cannulas **800, 900, 1000** may also be particularly well-suited for delivering stem cells to the brain tissue of a patient. In particular, the cannulas **800, 900, 1000** may be well-suited for implanting stem cells into the brain tissue of a patient. The cannulas **800, 900, 1000** may be used in the same manner as the cannula **700** to dispense stem cells into a patient.

As discussed herein, insertion of the surgical cannula **100** (or any other surgical, e.g., delivery, cannula) can be tracked in near real time by reference to a void in the patient tissue caused by the cannula **100** and reflected in the MR image. In some embodiments, one or more MRI-visible fiducial markers may be provided on the surgical cannula **100,** MR scanned and processed, and displayed on the UI. In some embodiments, the surgical cannula **100** may itself be formed of an MRI-visible material, MR scanned and processed, and displayed on the UI.

According to some embodiments, the surgical cannula may include an embedded intrabody MRI antenna that is configured to pick-up MRI signals in local tissue during an MRI procedure. The MRI antenna can be configured to reside on a distal end portion of the surgical cannula. In some embodiments, the antenna has a focal length or signal-receiving length of between about 1-5 cm, and typically is configured to have a viewing length to receive MRI signals from local tissue of between about 1-2.5 cm. The MRI antenna can be formed as comprising a coaxial and/or triaxial antenna. However, other antenna configurations can be used, such as, for example, a whip antenna, a coil antenna, a loopless antenna, and/or a looped antenna. *See, e.g.,* U.S. Patent Nos. 5,699,801; 5,928,145; 6,263,229; 6,606,513; 6,628,980; 6,284,971; 6,675,033; and 6,701176. *See also* U.S. Patent Application Publication Nos. 2003/0050557; 2004/0046557; and 2003/0028095.

Surgical cannulas **600, 700, 800, 900, 1000** as described herein may be used with a stereotactic frame or without a stereotactic frame.

While the surgical cannulas **600, 700, 800, 900, 1000** have been identified herein as delivery cannulas and methods for delivering a substance to a patient have been described, in accordance with some embodiments of the invention, the surgical cannulas can be used to withdraw a substance (*e.g*., spinal fluid) from a patient. Thus, it will be appreciated that surgical cannulas and methods as disclosed herein can be used to transfer a substance into and/or from a patient.

While the surgical cannulas **600, 700, 800, 900, 1000** have been described herein with reference to MRI-guided insertion and infusion procedures, in some embodiments the cannulas can be used in procedures without MRI guidance, such as CT-guided systems and may use other materials than described above.

While the surgical cannulas **600, 700, 800, 900, 1000** have been described in use with a trajectory guide **50b,** the cannulas may be used with other types of trajectory guidance or stereotactic frames or without a stereotactic frame or trajectory guide.

The surgical cannulas **600, 700, 800, 900, 1000** as depicted in **FIGS. 8A-****12B**may typically be employed for acute treatments. However, the systems, cannulas, methods and procedures described herein may likewise be used a chronic delivery cannula or catheter. For example, the delivery cannulas **600, 700, 800, 900, 1000** may be installed on a patient for chronic delivery as described in PCT Published Patent Application No. WO 2011/130107 A2 (Attorney Docket No. 9450-75WO).

According to some embodiments, the substance delivered via the delivery cannula includes radioactive objects such as radioactive seeds. In this event, the delivery cannula may include a suitable radiation shield or shielding material in order to reduce or prevent the exposure of tissue outside the target region to radiation from the radioactive objects.

The system **10** may also include a decoupling/tuning circuit that allows the system to cooperate with an MRI scanner **20** and filters and the like. *See, e.g.,* U.S. Patent Nos. 6,701,176; 6,904,307 and U.S. Patent Application Publication No. 2003/0050557.

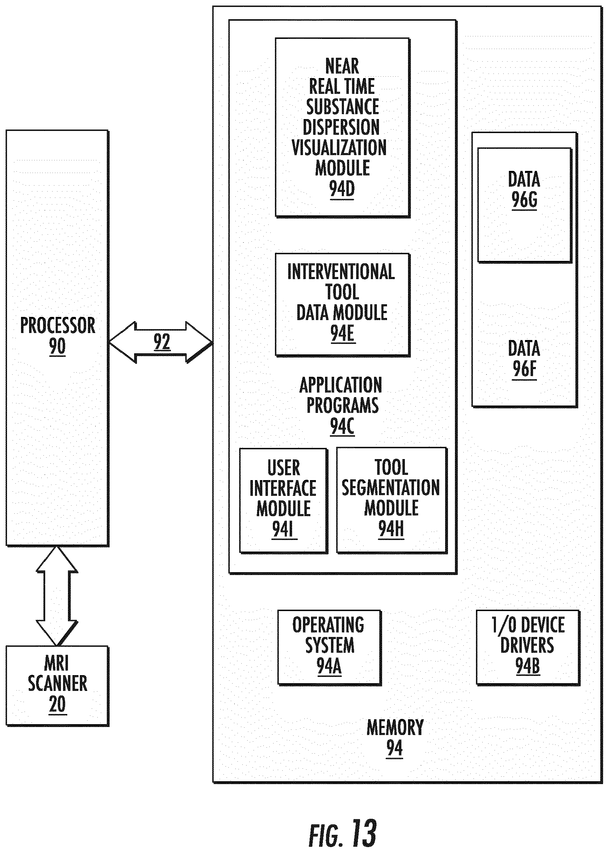

The system **10** can include circuits and/modules that can comprise computer program code used to automatically or semi-automatically carry out operations to generate visualizations and provide output to a user to facilitate MRI-guided diagnostic and therapy procedures. **FIG. 13** is a schematic illustration of a circuit or data processing system that can be used with the system **10.** The circuits and/or data processing systems may be incorporated in one or more digital signal processors in any suitable device or devices. The processor **90** communicates with an MRI scanner **20** and with memory **94** via an address/data bus **92.** The processor **90** can be any commercially available or custom microprocessor. The memory **94** is representative of the overall hierarchy of memory devices containing the software and data used to implement the functionality of the data processing system. The memory **94** can include, but is not limited to, the following types of devices: cache, ROM, PROM, EPROM, EEPROM, flash memory, SRAM, and DRAM.

The memory **94** may include several categories of software and data used in the data processing system: the operating system **94A;** the application programs **94C;** the input/output (I/O) device drivers **94B;** and data **94F.** The data **94F** can also include predefined characteristics of different surgical tools and patient image data **94G.** The application programs **94C** can include a Near Real-Time Substance Dispersion Visualization Module **94D,** Interventional Tool Data Module **94E,** a Tool Segmentation Module **94H** (such as segmentation modules for a targeting cannula, a trajectory guide frame and/or base, and a delivery cannula), and a workflow group User Interface Module **941** (that facilitates user actions and provides guidance to obtain a desired trajectory or a desired drug dispersion pattern, such as physical adjustments to achieve same).

As will be appreciated by those of skill in the art, the operating systems **94A** may be any operating system suitable for use with a data processing system, such as OS/2, AIX, DOS, OS/390 or System390 from International Business Machines Corporation, Armonk, NY, Windows CE, Windows NT, Windows95, Windows98, Windows2000 or other Windows versions from Microsoft Corporation, Redmond, WA, Unix or Linux or FreeBSD, Palm OS from Palm, Inc., Mac OS from Apple Computer, LabView, or proprietary operating systems. The I/O device drivers **94C** typically include software routines accessed through the operating system **94A** by the application programs **94C** to communicate with devices such as I/O data port(s), data storage **94F** and certain memory **94** components. The application programs **94C** are illustrative of the programs that implement the various features of the data processing system and can include at least one application, which supports operations according to embodiments of the present disclosure. Finally, the data **94F** represents the static and dynamic data used by the application programs **94C,** the operating system **94A,** the I/O device drivers **94C,** and other software programs that may reside in the memory **94.**

While the present disclosure is illustrated, for example, with reference to the Modules **94C, 94D, 94E, 94H, 941** being application programs in **FIG. 13****,** as will be appreciated by those of skill in the art, other configurations may also be utilized while still benefiting from the teachings of the present disclosure. For example, the Modules **94C, 94D, 94E, 94H, 941** and/or may also be incorporated into the operating system **94A,** the I/O device drivers **94B** or other such logical division of the data processing system. Thus, the present disclosure should not be construed as limited to the configuration of **FIG. 13****,** which is intended to encompass any configuration capable of carrying out the operations described herein. Further, one or more of modules, *i.e.,* Modules **94C, 94D, 94E, 94H, 941** can communicate with or be incorporated totally or partially in other components, such as a workstation, an MRI scanner, an interface device. Typically, the workstation **30** will include the modules **94C, 94D, 94E, 94H, 941** and the MR scanner with include a module that communicates with the workstation 30 and can push image data thereto.

The I/O data port can be used to transfer information between the data processing system, the circuit **30c** or workstation **30,** the MRI scanner **20,** and another computer system or a network (*e.g*., the Internet) or to other devices controlled by or in communication with the processor. These components may be conventional components such as those used in many conventional data processing systems, which may be configured in accordance with the present disclosure to operate as described herein.

It is noted that any one or more aspects or features described with respect to one embodiment, may be incorporated in a different embodiment although not specifically described relative thereto. That is, all embodiments and/or features of any embodiment can be combined in any way and/or combination.

Other systems, methods, and/or computer program products according to embodiments of the present disclosure will be or become apparent to one with skill in the art upon review of the following drawings and detailed description.

## Claims

1. A cannula (600, 900) for transferring a substance to and/or from a patient, the cannula comprising:
a rigid, tubular support sleeve (610, 910) defining a support sleeve lumen extending from a first opening at a proximal end of the support sleeve to a second opening at a distal end (611A) of the support sleeve, wherein the support sleeve (610, 910) comprises a rigid, MRI-compatible material;
a transfer tube (630, 930) disposed in the support sleeve lumen and extending beyond the distal end (631A) of the support sleeve to a distal end of the transfer tube, the transfer tube (630, 930) defining a transfer tube lumen terminating at an opening at the distal end of the transfer tube; and
a conformal polymeric sleeve (640, 940) including;
a proximal section (642) surrounding at least a portion of the support sleeve (610, 910); and
a transitional section (644, 944) connected to the proximal section (642) and surrounding at least a portion of the transfer tube (630, 930);
wherein:
the transitional section begins at the distal end (611A) of the support sleeve (610, 910) and extends from there in a direction toward the distal end (631A) of the transfer tube and over the transfer tube (630, 930); and
the transitional section (644, 944) tapers inwardly in the direction toward the distal end of the transfer tube (630, 930).

2. The cannula of Claim 1 wherein the transitional section (644, 944) is frustoconical.

3. The cannula of Claim 1 or 2 wherein the transitional section (644, 944) extends from the distal end (611A) of the support sleeve (610, 910) toward the distal end (631A) of the transfer tube (630, 930) an axial distance in the range of from about 0.1 mm to 5 mm.

4. The cannula of any one of the preceding claims wherein the transfer tube (930) is adhered by adhesive to an inner surface of the support sleeve (910).

5. The cannula of Claim 4 wherein:
the conformal polymeric sleeve (940) defines an annular void (V9) between an outer surface of the transfer tube (930) and the transitional section (944) of the conformal polymeric sleeve adjacent the distal end of the support sleeve; and
the cannula includes an adhesive (G9) at least partially filling the annular void (V9).

6. The cannula of Claim 4 or 5 wherein the conformal polymeric sleeve (940) extends to the distal end (931A) of the transfer tube (930).

7. The cannula of Claim 4 or 5 wherein the conformal polymeric sleeve (1040) has a distal end (1041A) located axially between the distal end (101 1A) of the support sleeve (1010) and the distal end (1031A) of the transfer tube (1030).

8. The cannula of any one of Claims 1 to 3 including an inner sleeve (620) disposed in the support sleeve lumen and extending beyond the distal end of the support sleeve (610) to a distal end of the inner sleeve, the inner sleeve (620) defining an inner sleeve lumen, and wherein:
the transfer tube (630) is disposed in the inner sleeve lumen and extends to or beyond the distal end of the inner sleeve to the distal end of the transfer tube (630);
the conformal polymeric sleeve (640) surrounds at least a portion of the inner sleeve (620);
the transitional section (644) extends from the distal end of the support sleeve (610) and over the inner sleeve (620) in a direction toward the distal end of the inner sleeve (620); and
the transitional section (644) tapers inwardly in the direction toward the distal end of the inner sleeve (620).

9. The cannula of Claim 8 wherein:
the conformal polymeric sleeve (640) defines an annular void (V6) between an outer surface of the inner sleeve (620) and the transitional section (644) of the conformal polymeric sleeve (640) adjacent the distal end of the support sleeve (610); and
the cannula includes an adhesive (G1) at least partially filling the annular void (V6).

10. The cannula of Claim 8 or 9 wherein the conformal polymeric sleeve (640) extends to the distal end of the inner sleeve (620).

11. The cannula of Claim 10 wherein the conformal polymeric sleeve (640) covers a terminal end face of the inner sleeve (620) at the distal end of the inner sleeve.

12. The cannula of Claim 10 or 11 wherein the transfer tube (630) extends axially beyond the inner sleeve (620) in the direction of the distal end of the transfer tube (630).

13. The cannula of any one of Claims 8 to 11 wherein the distal end of the inner sleeve (620) is coterminous with the distal end of the transfer tube (630).

14. The cannula of any one of Claims 8 to 11 wherein:
the transfer tube (630) extends beyond the distal end of the inner sleeve (620);
an exterior surface of the cannula has at least first, second and third co-axially disposed segments, an outer diameter of the second segment being greater than an outer diameter of the first segment, and an outer diameter of the third segment being greater than the outer diameter of the second segment;
the second segment extends between and adjoins each of the first and third segments;
the first segment extends from the distal end (631A) of the transfer tube (630) to the distal end (621A) of the inner sleeve (620);
the second segment extends from the distal end (621A) of the inner sleeve (620) to a distal end (641A) of the transitional section (644) of the conformal polymeric sleeve (640);
the third segment extends from a proximal end of the transitional section (644) toward the proximal end of the support sleeve (640);
a terminal end face (620B) of the inner sleeve forms a sharp step about the transfer tube (630) between the first segment and the second segment, the terminal end face (620B) of the inner sleeve projecting radially outwardly beyond the outer diameter of the first segment; and
the transitional section (644) defines a second step between the second segment and the third segment, the second step having a smooth rise from the outer diameter of the second segment to the outer diameter of the third segment.

15. The cannula of any one of Claims 8 to 14 wherein:
the inner sleeve (620) is adhered with adhesive to an inner surface of the tubular support sleeve (610); and
the inner sleeve (620) is adhered with adhesive to an outer surface of the transfer tube (630).

## Patentansprüche

1. Eine Kanüle (600, 900) zum Transferieren einer Substanz in einen und/oder aus einem Patienten, wobei die Kanüle Folgendes beinhaltet:
eine starre, rohrförmige Stützhülse (610, 910), die ein Stützhülsenlumen definiert, das sich von einer ersten Öffnung an einem proximalen Ende der Stützhülse zu einer zweiten Öffnung an einem distalen Ende (611A) der Stützhülse erstreckt, wobei die Stützhülse (610, 910) ein starres, MRT-kompatibles Material beinhaltet;
ein Transferrohr (630, 930), das in dem Stützhülsenlumen angeordnet ist und sich über das distale Ende (631A) der Stützhülse hinaus zu einem distalen Ende des Transferrohrs erstreckt, wobei das Transferrohr (630, 930) ein Transferrohrlumen definiert, das an einer Öffnung an dem distalen Ende des Transferrohrs endet; und
eine konforme Polymerhülse (640, 940), die Folgendes beinhaltet:
einen proximalen Abschnitt (642), der mindestens einen Anteil der Stützhülse (610, 910) umgibt; und
einen Übergangsabschnitt (644, 944), der mit dem proximalen Abschnitt (642) verbunden ist und mindestens einen Anteil des Transferrohrs (630, 930) umgibt;
wobei:
der Übergangsabschnitt an dem distalen Ende (611A) der Stützhülse (610, 910) beginnt und sich von dort in einer Richtung hin zu dem distalen Ende (631A) des Transferrohrs und über das Transferrohr (630, 930) erstreckt; und
der Übergangsabschnitt (644, 944) sich in der Richtung hin zu dem distalen Ende des Transferrohrs (630, 930) nach innen verjüngt.

2. Kanüle gemäß Anspruch 1, wobei der Übergangsabschnitt (644, 944) kegelstumpfförmig ist.

3. Kanüle gemäß Anspruch 1 oder 2, wobei sich der Übergangsabschnitt (644, 944) von dem distalen Ende (611A) der Stützhülse (610, 910) hin zu dem distalen Ende (631A) des Transferrohrs (630, 930) über eine axiale Entfernung im Bereich von etwa 0,1 mm bis 5 mm erstreckt.

4. Kanüle gemäß einem der vorhergehenden Ansprüche, wobei das Transferrohr (930) mit Klebstoff an eine innere Oberfläche der Stützhülse (910) geheftet ist.

5. Kanüle gemäß Anspruch 4, wobei:
die konforme Polymerhülse (940) einen ringförmigen Leerraum (V9) zwischen einer äußeren Oberfläche des Transferrohrs (930) und dem Übergangsabschnitt (944) der konformen Polymerhülse, angrenzend an das distale Ende der Stützhülse, definiert, und
die Kanüle einen Klebstoff (G9) umfasst, der den ringförmigen Leerraum (V9) mindestens teilweise füllt.

6. Kanüle gemäß Anspruch 4 oder 5, wobei sich die konforme Polymerhülse (940) zu dem distalen Ende (931A) des Transferrohrs (930) erstreckt.

7. Kanüle gemäß Anspruch 4 oder 5, wobei die konforme Polymerhülse (1040) ein distales Ende (1041A) aufweist, das axial zwischen dem distalen Ende (1011A) der Stützhülse (1010) und dem distalen Ende (1031A) des Transferrohrs (1030) liegt.

8. Kanüle gemäß einem der Ansprüche 1 bis 3, die eine Innenhülse (620) umfasst, die in dem Stützhülsenlumen angeordnet ist und sich über das distale Ende der Stützhülse (610) hinaus zu einem distalen Ende der Innenhülse erstreckt, wobei die Innenhülse (620) ein Innenhülsenlumen definiert, und wobei:
das Transferrohr (630) in dem Innenhülsenlumen angeordnet ist und sich zu dem oder über das distale Ende der Innenhülse hinaus zu dem distalen Ende des Transferrohrs (630) erstreckt;
die konforme Polymerhülse (640) mindestens einen Anteil der Innenhülse (620) umgibt;
der Übergangsabschnitt (644) sich von dem distalen Ende der Stützhülse (610) und über die Innenhülse (620) in einer Richtung hin zu dem distalen Ende der Innenhülse (620) erstreckt; und
der Übergangsabschnitt (644) sich in der Richtung hin zu dem distalen Ende der Innenhülse (620) nach innen verjüngt.

9. Kanüle gemäß Anspruch 8, wobei:
die konforme Polymerhülse (640) einen ringförmigen Leerraum (V6) zwischen einer äußeren Oberfläche der Innenhülse (620) und dem Übergangsabschnitt (644) der konformen Polymerhülse (640), angrenzend an das distale Ende der Stützhülse (610), definiert, und
die Kanüle einen Klebstoff (G1) umfasst, der den ringförmigen Leerraum (V6) mindestens teilweise füllt.

10. Kanüle gemäß Anspruch 8 oder 9, wobei sich die konforme Polymerhülse (640) zu dem distalen Ende der Innenhülse (620) erstreckt.

11. Kanüle gemäß Anspruch 10, wobei die konforme Polymerhülse (640) eine abschließende Endfläche der Innenhülse (620) an dem distalen Ende der Innenhülse bedeckt.

12. Kanüle gemäß Anspruch 10 oder 11, wobei sich das Transferrohr (630) axial über die Innenhülse (620) hinaus in der Richtung des distalen Endes des Transferrohrs (630) erstreckt.

13. Kanüle gemäß einem der Ansprüche 8 bis 11, wobei das distale Ende der Innenhülse (620) an der gleichen Stelle wie das distale Ende des Transferrohrs (630) endet.

14. The Kanüle gemäß einem der Ansprüche 8 bis 11, wobei:
das Transferrohr (630) sich über das distale Ende der Innenhülse (620) hinaus erstreckt;
eine äußere Oberfläche der Kanüle mindestens erste, zweite und dritte koaxial angeordnete Segmente aufweist, wobei ein Außendurchmesser des zweiten Segments größer als ein Außendurchmesser des ersten Segments ist und ein Außendurchmesser des dritten Segments größer als der Außendurchmesser des zweiten Segments ist;
das zweite Segment sich zwischen dem ersten und dritten Segment erstreckt und an jedes von ihnen anstößt;
das erste Segment sich von dem distalen Ende (631A) des Transferrohrs (630) zu dem distalen Ende (621A) der Innenhülse (620) erstreckt;
das zweite Segment sich von dem distalen Ende (621A) der Innenhülse (620) zu einem distalen Ende (641A) des Übergangsabschnitts (644) der konformen Polymerhülse (640) erstreckt;
das dritte Segment sich von einem proximalen Ende des Übergangsabschnitts (644) hin zu dem proximalen Ende der Stützhülse (640) erstreckt;
eine abschließende Endfläche (620B) der Innenhülse eine scharfkantige Stufe um das Transferrohr (630) herum zwischen dem ersten Segment und dem zweiten Segment bildet, wobei die abschließende Endfläche (620B) der Innenhülse radial über den Außendurchmesser des ersten Segments hinaus nach außen vorsteht; und
der Übergangsabschnitt (644) eine zweite Stufe zwischen dem zweiten Segment und dem dritten Segment definiert, wobei die zweite Stufe eine glatte Steigungshöhe von dem Außendurchmesser des zweiten Segments zu dem Außendurchmesser des dritten Segments aufweist.

15. Kanüle gemäß einem der Ansprüche 8 bis 14, wobei:
die Innenhülse (620) mit Klebstoff an eine innere Oberfläche der rohrförmigen Stützhülse (610) geheftet ist; und
die Innenhülse (620) mit Klebstoff an eine äußere Oberfläche des Transferrohrs (630) geheftet ist.

## Revendications

1. Canule (600, 900) pour le transfert d'une substance vers et/ou depuis un patient, la canule comprenant :
un manchon de support (610, 910) tubulaire rigide définissant une lumière de manchon de support s'étendant d'une première ouverture à une extrémité proximale du manchon de support à une deuxième ouverture à une extrémité distale (611A) du manchon de support, dans laquelle le manchon de support (610, 910) comprend un matériau compatible IRM rigide ;
un tube de transfert (630, 930) disposé dans la lumière de manchon de support et s'étendant au-delà de l'extrémité distale (631A) du manchon de support jusqu'à une extrémité distale du tube de transfert, le tube de transfert (630, 930) définissant une lumière de tube de transfert se terminant au niveau d'une ouverture à l'extrémité distale du tube de transfert ; et
un manchon polymère conforme (640, 940) comportant ;
une section proximale (642) entourant au moins une portion du manchon de support (610, 910) ; et
une section de transition (644, 944) raccordée à la section proximale (642) et entourant au moins une portion du tube de transfert (630, 930) ;
dans laquelle :
la section de transition commence à l'extrémité distale (611A) du manchon de support (610, 910) et s'étend depuis celle-ci dans une direction vers l'extrémité distale (631A) du tube de transfert et au-dessus du tube de transfert (630, 930) ; et
la section de transition (644, 944) se rétrécit vers l'intérieur dans la direction vers l'extrémité distale du tube de transfert (630, 930).

2. Canule selon la revendication 1 dans laquelle la section de transition (644, 944) est tronconique.

3. Canule selon la revendication 1 ou 2 dans laquelle la section de transition (644, 944) s'étend depuis l'extrémité distale (611A) du manchon de support (610, 910) vers l'extrémité distale (631A) du tube de transfert (630, 930) sur une distance axiale dans la plage d'environ 0,1 mm à 5 mm.

4. Canule selon l'une quelconque des revendications précédentes dans laquelle le tube de transfert (930) est collé par adhésif à une surface interne du manchon de support (910).

5. Canule selon la revendication 4 dans laquelle :
le manchon polymère conforme (940) définit un vide annulaire (V9) entre une surface externe du tube de transfert (930) et la section de transition (944) du manchon polymère conforme adjacent à l'extrémité distale du manchon de support ; et
la canule comporte un adhésif (G9) remplissant au moins partiellement le vide annulaire (V9).

6. Canule selon la revendication 4 ou 5 dans laquelle le manchon polymère conforme (940) s'étend jusqu'à l'extrémité distale (931A) du tube de transfert (930).

7. Canule selon la revendication 4 ou 5 dans laquelle le manchon polymère conforme (1040) a une extrémité distale (1041A) située axialement entre l'extrémité distale (1011A) du manchon de support (1010) et l'extrémité distale (1031A) du tube de transfert (1030).

8. Canule selon l'une quelconque des revendications 1 à 3 comportant un manchon interne (620) disposé dans la lumière de manchon de support et s'étendant au-delà de l'extrémité distale du manchon de support (610) jusqu'à une extrémité distale du manchon interne, le manchon interne (620) définissant une lumière de manchon interne, et dans laquelle :
le tube de transfert (630) est disposé dans la lumière de manchon interne et s'étend jusqu'à l'extrémité distale du manchon interne ou au-delà de celle-ci jusqu'à l'extrémité distale du tube de transfert (630) ;
le manchon polymère conforme (640) entoure au moins une portion du manchon interne (620) ;
la section de transition (644) s'étend depuis l'extrémité distale du manchon de support (610) et au-dessus du manchon interne (620) dans une direction vers l'extrémité distale du manchon interne (620) ; et
la section de transition (644) se rétrécit vers l'intérieur dans la direction vers l'extrémité distale du manchon interne (620).

9. Canule selon la revendication 8 dans laquelle :
le manchon polymère conforme (640) définit un vide annulaire (V6) entre une surface externe du manchon interne (620) et la section de transition (644) du manchon polymère conforme (640) adjacent à l'extrémité distale du manchon de support (610) ; et
la canule comporte un adhésif (G1) remplissant au moins partiellement le vide annulaire (V6).

10. Canule selon la revendication 8 ou 9 dans laquelle le manchon polymère conforme (640) s'étend jusqu'à l'extrémité distale du manchon interne (620).

11. Canule selon la revendication 10 dans laquelle le manchon polymère conforme (640) couvre une face d'extrémité terminale du manchon interne (620) à l'extrémité distale du manchon interne.

12. Canule selon la revendication 10 ou 11 dans laquelle le tube de transfert (630) s'étend axialement au-delà du manchon interne (620) dans la direction de l'extrémité distale du tube de transfert (630).

13. Canule selon l'une quelconque des revendications 8 à 11 dans laquelle l'extrémité distale du manchon interne (620) est coïncidente avec l'extrémité distale du tube de transfert (630).

14. Canule selon l'une quelconque des revendications 8 à 11 dans laquelle :
le tube de transfert (630) s'étend au-delà de l'extrémité distale du manchon interne (620) ;
une surface extérieure de la canule a au moins des premier, deuxième et troisième segments disposés coaxialement, un diamètre externe du deuxième segment étant plus grand qu'un diamètre externe du premier segment, et un diamètre externe du troisième segment étant plus grand que le diamètre externe du deuxième segment ;
le deuxième segment s'étend entre chacun des premier et troisième segments et les jouxte ;
le premier segment s'étend de l'extrémité distale (631A) du tube de transfert (630) à l'extrémité distale (621A) du manchon interne (620) ;
le deuxième segment s'étend de l'extrémité distale (621A) du manchon interne (620) à une extrémité distale (641A) de la section de transition (644) du manchon polymère conforme (640) ;
le troisième segment s'étend depuis une extrémité proximale de la section de transition (644) vers l'extrémité proximale du manchon de support (640) ;
une face d'extrémité terminale (620B) du manchon interne forme un gradin net autour du tube de transfert (630) entre le premier segment et le deuxième segment, la face d'extrémité terminale (620B) du manchon interne faisant saillie radialement vers l'extérieur au-delà du diamètre externe du premier segment ; et
la section de transition (644) définit un deuxième gradin entre le deuxième segment et le troisième segment, le deuxième gradin ayant une légère remontée du diamètre externe du deuxième segment au diamètre externe du troisième segment.

15. Canule selon l'une quelconque des revendications 8 à 14 dans laquelle :
le manchon interne (620) est collé par adhésif à une surface interne du manchon de support tubulaire (610) ; et
le manchon interne (620) est collé par adhésif à une surface externe du tube de transfert (630).
